# EUROPEAN PATENT SPECIFICATION

(11) **EP 2 706 120 B1**
(45) Date of publication and mention of the grant of the patent: **20.04.2016**
(21) Application number: 12183209.1
(22) Date of filing: 05.09.2012
(51) Int. Cl.: C12Q 1/00, C12Q 1/26, C12N 9/00, C12N 15/00, G01N 27/00

(54) **Biosensor comprising a mtr-gene construct**
Biosensor mit einem MTR-Gen-Konstrukt
Biocapteur comprenant une construction de gène mtr

(43) Date of publication of application: 12.03.2014
(73) Proprietor: Karlsruher Institut für Technologie, 76131 Karlsruhe (DE)
(72) Inventor: Buecking, Clemens, 79104 Freiburg (DE); Golitsch, Frederik, 76133 Karlsruhe (DE); Gescher, Johannes, 76133 Karlsruhe (DE)
(74) Representative: Elbel, Michaela

(56) References cited:
- WO-A2-2012/168743
- JENSEN HEATHER M ET AL: "Engineering of a synthetic electron conduit in living cells", PROCEEDINGS OF THE NATIONAL ACADEMY OF SCIENCES OF THE UNITED STATES OF AMERICA, vol. 107, no. 45, November 2010 (2010-11), pages 19213-19218, XP002714502, ISSN: 0027-8424
- DAN COURSOLLE ET AL: "Reconstruction of Extracellular Respiratory Pathways for Iron(III) Reduction in Shewanella Oneidensis Strain MR-1", FRONTIERS IN MICROBIOLOGY, vol. 3, 1 January 2012 (2012-01-01), XP055083220, DOI: 10.3389/fmicb.2012.00056
- FREDERIK GOLITSCH ET AL: "Proof of principle for an engineered microbial biosensor based on Shewanella oneidensis outer membrane protein complexes", BIOSENSORS AND BIOELECTRONICS, vol. 47, 1 September 2013 (2013-09-01), pages 285-291, XP055082945, ISSN: 0956-5663, DOI: 10.1016/j.bios.2013.03.010
- C. BUCKING ET AL: "Outer-membrane cytochrome-independent reduction of extracellular electron acceptors in Shewanella oneidensis", MICROBIOLOGY, vol. 158, no. Pt_8, 5 April 2012 (2012-04-05), pages 2144-2157, XP055083033, ISSN: 1350-0872, DOI: 10.1099/mic.0.058404-0
- M. SCHICKLBERGER ET AL: "Involvement of the Shewanella oneidensis Decaheme Cytochrome MtrA in the Periplasmic Stability of the -Barrel Protein MtrB", APPLIED AND ENVIRONMENTAL MICROBIOLOGY, vol. 77, no. 4, 17 December 2010 (2010-12-17), pages 1520-1523, XP055083037, ISSN: 0099-2240, DOI: 10.1128/AEM.01201-10
- CLEMENS BÜCKING ET AL: "Involvement and specificity of Shewanella oneidensis outer membrane cytochromes in the reduction of soluble and solid-phase terminal electron acceptors", FEMS MICROBIOLOGY LETTERS, WILEY-BLACKWELL PUBLISHING LTD, GB, vol. 306, no. 2, 1 May 2010 (2010-05-01), pages 144-151, XP002683902, ISSN: 0378-1097, DOI: 10.1111/J.1574-6968.2010.01949.X [retrieved on 2010-03-30]

### SEQUENCE LISTING

<110> Karlsruher Insitut für Technologie
<120> Biosensor comprising a mtr-gene construct
<130> T30043
<160> 5
<170> PatentIn version 3.3
<210> 1
   <211> 48
   <212> DNA
   <213> artificial
<220>
   <223> Primer sequence
<400> 1
   atgattacga attcgagctc ggtacccggg ctgaccgcga atggtgag 48
<210> 2
   <211> 51
   <212> DNA
   <213> artificial
<220>
   <223> primer sequence
<400> 2
   tcttcataat aggcttccca atttgtccca ttatttttcg aactgcgggt g 51
<210> 3
   <211> 19
   <212> DNA
   <213> artificial
<220>
   <223> primer sequence
<400> 3
   tgggacaaat tgggaagcc 19
<210> 4
   <211> 50
   <212> DNA
   <213> artificial
<220>
   <223> primer sequence
<400> 4
   cggccagtgc caagcttgca tgcctgcagg gcaacatcgg cattgtcatg 50
<210> 5
   <211> 6912
   <212> DNA
   <213> artificial
<220>
   <223> DNA construct
<220>
   <221> araC
   <222> (271)..(1200)
<220>
   <221> mtrFstrep
   <222> (1521)..(3437)
<220>
   <221> strep-tag
   <222> (3438)..(3464)
<220>
   <221> mtrA
   <222> (3488)..(4489)
<220>
   <221> mtrB
   <222> (4502)..(6595)
<400> 5
   cttaccattt atccctacag aatcattcct ctttacagtt aagtcgacca aatatgttaa      60
   gcagctgtca caaacctgtt ctaatattaa tcaactaaat tagtgatgtc gaaagcttga     120
   aaaataacaa ataaaacgca ctcgggaaaa atggacatgg atattggttt aaagttcaac     180
   agcataactc aaatgtcaaa tggacgaagc agggattctg caaaccctat gctactccgt     240
   caagccgtca attgtctgat tcgttaccaa ttatgacaac ttgacggcta catcattcac     300
   tttttcttca caaccggcac ggaactcgct cgggctggcc ccggtgcatt ttttaaatac     360
   ccgcgagaaa tagagttgat cgtcaaaacc aacattgcga ccgacggtgg cgataggcat     420
   ccgggtggtg ctcaaaagca gcttcgcctg gctgatacgt tggtcctcgc gccagcttaa     480
   gacgctaatc cctaactgct ggcggaaaag atgtgacaga cgcgacggcg acaagcaaac     540
   atgctgtgcg acgctggcga tatcaaaatt gctgtctgcc aggtgatcgc tgatgtactg     600
   acaagcctcg cgtacccgat tatccatcgg tggatggagc gactcgttaa tcgcttccat     660
   gcgccgcagt aacaattgct caagcagatt tatcgccagc agctccgaat agcgcccttc     720
   cccttgcccg gcgttaatga tttgcccaaa caggtcgctg aaatgcggct ggtgcgcttc     780
   atccgggcga aagaaccccg tattggcaaa tattgacggc cagttaagcc attcatgcca     840
   gtaggcgcgc ggacgaaagt aaacccactg gtgataccat tcgcgagcct ccggatgacg     900
   accgtagtga tgaatctctc ctggcgggaa cagcaaaata tcacccggtc ggcaaacaaa     960
   ttctcgtccc tgatttttca ccaccccctg accgcgaatg gtgagattga gaatataacc    1020
   tttcattccc agcggtcggt cgataaaaaa atcgagataa ccgttggcct caatcggcgt    1080
   taaacccgcc accagatggg cattaaacga gtatcccggc agcaggggat cattttgcgc    1140
   ttcagccata cttttcatac tcccgccatt cagagaagaa accaattgtc catattgcat    1200
   cagacattgc cgtcactgcg tcttttactg gctcttctcg ctaaccaaac cggtaacccc 1260
   gcttattaaa agcattctgt aacaaagcgg gaccaaagcc atgacaaaaa cgcgtaacaa 1320
   aagtgtctat aatcacggca gaaaagtcca cattgattat ttgcacggcg tcacactttg 1380
   ctatgccata gcatttttat ccataagatt agcggatcct acctgacgct ttttatcgca 1440
   actctctact gtttctccat acccgttttt ttgggctaga aataattttg tttaacttta 1500
   agaaggagat atacataccc atgaataagt ttgcaagctt taccacgcaa tacagtctga 1560
   tgctgctcat tgccacgcta ctctctgcct gtggaggcag tgatggtgat gatggctcac 1620
   ccggcgagcc aggtaaacct ccggcaatga caatcagcag cctaaatatc agtgtagata 1680
   aagtcgccat cagcgatggt attgcccaag ttgattatca agtcagcaac caagaaaacc 1740
   aagccgtagt gggtattcct tccgccacct ttatcgctgc gcaattactg cctcaaggcg 1800
   ctacaggcgc tggcaatagt agtgagtggc agcattttac ctcagaaacc tgcgccgctt 1860
   catgtcccgg cactttcgtc gatcataaaa acggccatta tagttatcgc tttagtgcga 1920
   cattcaacgg catgaatggg gtgacatttc taagtgatgc cacccaacgc ttagtgataa 1980
   aaatcggtgg tgatgcgctc gccgatggca ctgtactccc tataaccaac caacattatg 2040
   attggcagtc ctcaggcaat atgctggcct atacccgtaa cttagtctcg atcgacactt 2100
   gtaatagttg ccatagtaat ttggctttcc atggaggacg ttataatcaa gttgaaacct 2160
   gtgtgacctg ccataacagt aaaaaagtca gcaatgccgc ggatattttc ccgcaaatga 2220
   tccacagtaa acatttaacc ggattccctc aatccatcag taattgccag acttgccatg 2280
   ctgataaccc tgatttggcc gatcgtcaaa attggtaccg agtaccgacc atggaagcct 2340
   gcggtgcatg tcatactcaa atcaatttcc ctgcgggtca aggccaccca gcgcaaacgg 2400
   ataatagcaa ttgcgttgcc tgtcacaatg cagattggac ggcaaacgtg cacagtaatg 2460
   cagctcaaac ctctgccttg gctcagttta atgcaagcat cagcagtgcc agtatggatg 2520
   ccaatggcac aatcacggtc gcggtgagcc taaccaaccc aaccacagga accgcttatg 2580
   ctgatagcgc cgataaatta aaatttatta gtgacttaag gatttatgct aactggggaa 2640
   ccagcttcga ctacagcagc cgttctgctc gctcgattag acttccggaa tcaaccccca 2700
   tagcaggaag caatggaaca tacagctaca atatttcagg tctcacagta cccgcaggta 2760
   ctgagtccga ccgtggcgga ttggccattc aaggtcgagt gtgcgcaaaa gatagtgtct 2820
   tagtggattg cagcaccgaa ctggcagaag tgcttgtgat caaatcaagt cacagttact 2880
   tcaatatgtc tgcattaacc accacaggca gacgcgaagt catcagtaat gcaaaatgtg 2940
   ctagctgcca tggcgatcag caattaaaca tccatggcgc ccgcaacgat ttagcgggtc 3000
   aatgtcagct ctgccacaat ccgaatatgc tcgccgacgc cacagcaacc aacccatcga 3060
   tgacatcttt tgattttaaa cagttaatcc acgggctcca tagcagccaa tttgcaggtt 3120
   ttgaagacct caattaccct gggaatatcg gtaattgcgc ccaatgccac atcaacgatt 3180
   cgacaggtat ctctactgta gccctcccct taaatgcggc cgttcaacct ctcgcgctta 3240
   acaatggcac cttcaccagt ccaattgccg ctgtatgtag caattgtcac tcaagtgatg 3300
   caactcaaaa tcatatgagg caacaaggtg cagtgtttgc cggaaccaaa gccgatgcaa 3360
   ccgcaggcac tgaaacctgt gcattttgcc acggacaagg cactgtcgcc gacgtactca 3420
   aagtccatcc aataaactgg agccacccgc agttcgaaaa ataatgggac aaattgggaa 3480
   gcctattatg aagaactgcc taaaaatgaa aaacctactg ccggcactta ccatcacaat 3540
   ggcaatgtct gcagttatgg cattagtcgt cacaccaaac gcttatgcgt cgaagtggga 3600
   tgagaaaatg acgccagagc aagtcgaagc caccttagat aagaagtttg ccgaaggcaa 3660
   ctactcccct aaaggcgccg attcttgctt gatgtgccat aagaaatccg aaaaagtcat 3720
   ggaccttttc aaaggtgtcc acggtgcgat tgactcctct aagagtccaa tggctggcct 3780
   gcaatgtgag gcatgccacg gcccactggg tcagcacaac aaaggcggca acgagccgat 3840
   gatcactttt ggtaagcaat caaccttaag tgccgacaag caaaacagcg tatgtatgag 3900
   ctgtcaccaa gacgataagc gtatgtcttg gaatggcggt caccatgaca atgccgatgt 3960
   tgcttgtgct tcttgtcacc aagtacacgt cgcaaaagat cctgtgttat ctaaaaacac 4020
   ggaaatggaa gtctgtacta gctgccatac aaagcaaaaa gcggatatga ataaacgctc 4080
   aagtcaccca ctcaaatggg cacaaatgac ctgtagcgac tgtcacaatc cccatgggag 4140
   catgacagat tccgatctta acaagcctag cgtgaatgat acctgttatt cctgtcacgc 4200
   cgaaaaacgc ggcccaaaac tttgggagca tgcacccgtc actgagaatt gtgtcacttg 4260
   ccacaatcct cacggtagtg tgaatgacgg tatgctgaaa acccgtgcgc cacagctatg 4320
   tcagcaatgt cacgccagcg atggccacgc cagcaacgcc tacttaggta acactggatt 4380
   aggttcaaat gtcggtgaca atgcctttac tggtggaaga agctgcttaa attgccatag 4440
   tcaggttcat ggttctaacc atccatctgg caagctatta cagcgctaag gagacgagaa 4500
   aatgaaattt aaactcaatt tgatcactct agcgttatta gccaacacag gcttggccgt 4560
   cgctgctgat ggttatggtc tagcgaatgc caatactgaa aaagtgaaat tatccgcatg 4620
   gagctgtaaa ggctgcgtcg ttgaaacggg cacatcaggc actgtgggtg tcggtgtcgg 4680
   ttataacagc gaagaggata ttcgctctgc caatgccttt ggtacatcca atgaagtggc 4740
   gggtaaattt gatgccgatt taaactttaa aggtgaaaag ggttatcgtg ccagtgttga 4800
   tgcttatcaa ctcggtatgg atggcggtcg cttagatgtc aatgcgggca aacaaggcca 4860
   gtacaacgtc aatgtgaact atcgccaaat tgctacctac gacagcaata gcgccctatc 4920
   gccctacgcg ggtattggtg gcaataacct cacgttaccg gataactgga taacagcagg 4980
   ttcaagcaac caaatgccac tcttgatgga cagcctcaat gccctcgaac tctcacttaa 5040
   acgtgagcgc acggggttgg gatttgaata tcaaggtgaa tccctgtgga gcacctatgt 5100
   taactacatg cgtgaagaga aaaccggctt aaaacaagcc tctggtagct tcttcaacca 5160
   atcgatgatg ttagcagagc cggtggatta caccactgac accattgaag cgggtgtcaa 5220
   actcaagggt gatcgttggt ttaccgcact cagttacaat gggtcaatat tcaaaaacga 5280
   atacaaccaa ttggactttg aaaatgcttt taaccccacc tttggtgctc aaacccaagg 5340
   tacgatggca ctcgatccgg ataaccagtc acacaccgtg tcgctgatgg gacagtacaa 5400
   cgatggcagc aacgcactgt cgggtcgtat tctgaccgga caaatgagcc aagatcaggc 5460
   gttagtgacg gataactacc gttatgctaa tcagctcaat accgatgccg tcgatgccaa 5520
   agtcgatcta ctgggtatga acctgaaagt cgttagcaaa gtgagcaatg atcttcgctt 5580
   aacaggtagt tacgattatt acgaccgtga caataatacc caagtagaag aatggactca 5640
   gatcagcatc aacaatgtca acggtaaggt ggcttataac accccttacg ataatcgtac 5700
   gcaacgcttt aaagttgccg cagattatcg cattacccgc gatatcaaac tcgatggtgg 5760
   ttatgacttc aaacgtgacc aacgtgatta tcaagaccgt gaaaccacgg atgaaaatac 5820
   cgtttgggcc cgtttacgtg taaacagctt cgatacttgg gacatgtggg taaaaggcag 5880
   ttacggtaac cgtgacggct cacaatacca agcgtctgaa tggacctctt ctgaaaccaa 5940
   cagcctgtta cgtaagtaca atctggctga ccgtgacaga actcaagtcg aagcacggat 6000
   cacccattcg ccattagaaa gcctgactat cgatgttggt gcccgttacg cgttagatga 6060
   ttataccgat actgtgattg gattaactga gtcaaaagac accagttatg atgccaacat 6120
   cagttatatg atcaccgctg acttactggc aaccgccttc tacaattacc aaaccattga 6180
   gtctgaacag gcgggtagca gcaattacag caccccaacg tggacaggct ttatagaaga 6240
   tcaggtagat gtggtcggtg caggtatcag ctacaacaat ctgctggaga acaagttacg 6300
   cctaggactg gactacacct attccaactc cgacagtaac actcaagtca gacaaggtat 6360
   cactggcgac tatggtgatt attttgccaa agtgcataac attaacttat acgctcaata 6420
   tcaagccacc gagaaactcg cgctgcgctt cgattacaaa attgagaact ataaggacaa 6480
   tgacgccgca aatgatatcg ccgttgatgg catttggaac gtcgtaggtt ttggtagtaa 6540
   cagccatgac tacaccgcac aaatgctgat gctgagcatg agttacaaac tctaatccat 6600
   ttgcctcata tgctcaaaag cccgcctctg cgggcttttt tatatcccgc agttttatat 6660
   ccagcagtga gtctcaatag cgcacaaata taggacggac aagggattaa tcctacgtaa 6720
   aaaatattga atgagatcag cctctaaagc aaaaagctat ctaaataacg tgagctcggg 6780
   taagtagagc cgcgaaatag gcatcttttc ggacctctct gcgttgctct gtctaaccat 6840
   agctcgctat ggcgtaccac tgagcgcctt gatatgcgca aaaatctgca cattcagtca 6900
   gaaaaaattc tt 6912

## Description

### Field of the Invention

The description relates to a nucleic acid construct for detecting a compound comprising a coding region and a regulatory element, which controls expression of the coding region depending on the compound. The invention relates to a method for detecting a compound in a sample. The invention further relates to a biosensor for detecting a compound comprising an electrode system, a device for measuring electron transfer in the electrode system, and a microorganism. Likewise, the invention relates to a biosensor for monitoring a fermentation process comprising at least one bio-electrochemical cell with a culture of a microorganism.

### Background of the Invention

Presently, the use of microbiological processes for technical and industrial applications is strongly increasing, including manufacturing recombinant products of biological or biochemical origin, microbial detoxification of waste or contaminated soil as well as generating power, e.g. through the production of biogas or H₂. Common to all these processes is the use of living microorganisms to carry out anabolic or catabolic actions. To do so, the microorganisms need to be kept under optimal conditions, which depend on the kind and species of microorganism used. In particular when employing them in technological or industrial processes, it has to be ensured that the microorganisms display an optimal growth and metabolism. Otherwise, the process becomes instable and inefficient. Therefore, microbiological processes need to be tightly controlled e.g. with respect to the growth and density of microorganisms, the availability of nutrients and the presence of inhibitory compounds in the culture.

Besides general parameters as temperature, oxygen level and pH, many microorganisms are sensitive to specific substances, which promote or repress their metabolism, either in general or with respect to specific pathways. Thus, the presence of specific substances within the microbiological culture can indicate the viability of the microorganisms in general as well as the productivity of specific pathways. For example, during biogas production an enrichment of long chain carbon acids is indicative for impaired fermentation and consequently low biogas production. In severe cases, high levels of long chain carbon acids can even cause the fermentation to break down entirely. This is because a variety of microorganisms involved in biogas production, in particular methanogenic archaea, are sensitive to high concentrations of acid. Therefore, the fermentation is usually controlled by regularly measuring the pH of the fermenter's content. However, measuring the pH does not distinguish individual kinds of acids, which is important in this context, since methanogenic microorganisms are able to catabolize formic acid and acetic acid but not carbon acids of longer chains. In addition, especially when feeding the fermenter with substrates comprising protein or fat, high concentrations of long chain carbon acids occur simultaneously with the production of ammonium compounds, which both considerably inhibit fermentation. However, due to the simultaneously increasing levels of acids and alkaline ammonium the overall pH within the fermenter may not be significantly altered.

Moreover, other compounds such as e.g. nutrients, minerals or phytochemicals cannot be detected indirectly by measuring pH or temperature.

Therefore, to specifically assess individual compounds or metabolites within a microbiological culture chromatographic, spectroscopic or spectrometric methods are employed. However, these are elaborate methods requiring specific technical equipment and therefore cannot be performed locally, e.g. at the site of fermentation. Instead, samples have to be taken from microbiological cultures and transported to specialized laboratories. Due to the necessary transport and the need of specific technological instruments, which can only be operated by qualified staff, the monitoring of microbiological cultures is particularly expensive.

Thus, fast and direct methods for determining specific compounds in microbiological cultures are needed. Likewise, easy to operate tools are needed, which can be used locally i.e. at the site of the microbiological culture.

### Summary of the Invention

In a first aspect, the description is directed to a nucleic construct for detecting a compound comprising a coding region and a regulatory element, which controls expression of the coding region depending on the compound, wherein the coding region comprises the genes *mtrA* or *mtrD;* and *mtrB* or *mtrE;* and *mtrC* or *mtrF.*

In a second aspect, the description is directed to a cell comprising such a nucleic acid.

In a further aspect, the invention is directed to a method for detecting a compound in a sample comprising providing a microorganism having a nucleic acid construct comprising the genes *mtrA, mtrB* and *mtrF* under a regulatory element depending on the compound, the microorganism being devoid of endogenous outer membrane associates cytochrome genes, contacting the microorganism with an electrode system such that the microorganism transfers electrons to the anode of the electrode system if the genes are expressed, adding a sample to the microorganism, and measuring the electron transfer to the electrode system, wherein the electron transfer to the electrode system indicates the absence, presence and/or concentration of the compound in the sample.

In a further aspect, the invention is directed to a biosensor for detecting a compound comprising an electrode system, a device for measuring electron transfer in the electrode system, and a microorganism having a nucleic acid construct comprising the genes *mtrA*, *mtrB* and *mtrF*, which transfers electrons to the electrode system if the genes are expressed, wherein the regulatory element is controlled by the compound and the microorganism is devoid of endogenous outer membrane associates cytochrome genes.

In a further aspect, the invention is directed to a biosensor for monitoring a fermentation process comprising at least one bio-electrochemical cell with a culture of a microorganism having a nucleic acid construct comprising the genes *mtrA*, *mtrB* and *mtrF* under a regulatory element such that the microorganism transfers electrons to an electrode of the bio-electrochemical cell if the genes are expressed, wherein the expression of the genes depends on a product or a substrate of the fermentation process and the microorganism is devoid of endogenous outer membrane associates cytochrome genes.

In a further aspect, the invention is directed to a biosensor for monitoring a fermentation process comprising two bio-electrochemical cells each with a culture of a microorganism having a nucleic acid construct comprising the genes *mtrA, mtrB* and *mtrF* under a regulatory element, such that the microorganism transfers electrons to an electrode of the bio-electrochemical cell if the genes are expressed, wherein the regulatory element of the first microorganism is a propionic acid dependent regulatory element and the regulatory element of the second microorganism is an acetic acid dependent regulatory element and wherein the microorganism is devoid of endogenous outer membrane associates cytochrome genes.

In a further aspect, the description is directed to the use of a nucleic acid construct for detecting a compound comprising a coding region and a regulatory element which controls expression of the coding region depending on the compound, wherein the coding region comprises the genes *mtrA* or *mtrD*; and *mtrB* or *mtrE*; and *mtrC* or *mtrF.*
In a further aspect, the invention relates to the use of a cell for detecting a compound comprising a nucleic acid construct with a coding region and a regulatory element, which controls expression of the coding region depending on the compound, wherein the coding region comprises the genes *mtrA*, *mtrB* and *mtrF* wherein the cell is devoid of endogenous outer membrane associates cytochrome genes.

### Description of the drawings

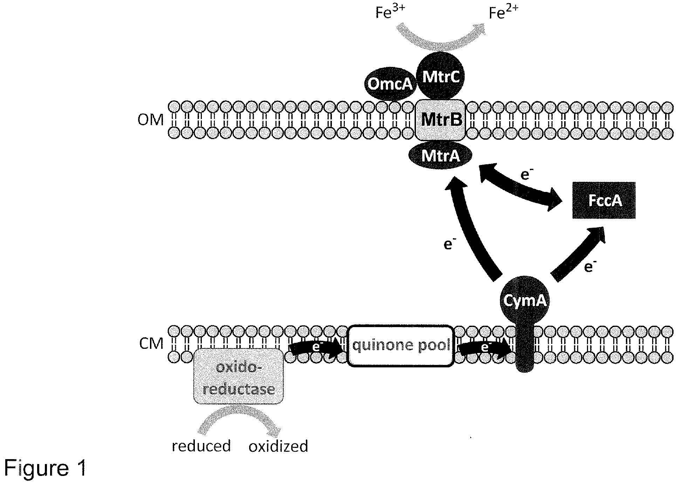

Figure 1 shows a schematic drawing of the extended electron transfer chain, transferring electrons from the cytoplasmic membrane through the periplasm and the outer membrane. Of the bacteria known to reduce extracellular electron acceptors, *Shewanella* and *Geobacter* were the most extensively studied ones. Using total genome sequencing and mutant analysis of S. *oneidensis* several proteins were identified which participate in the extended respiratory chain: MtrA, MtrB, MtrC, OmcA, and CymA. In addition, further genes were identified, which have electron transfer properties and thus may also participate in the extended respiratory chain. FccA: periplasmatic fumarate reductase; OM: outer membrane; CM: cytoplasmic membrane.

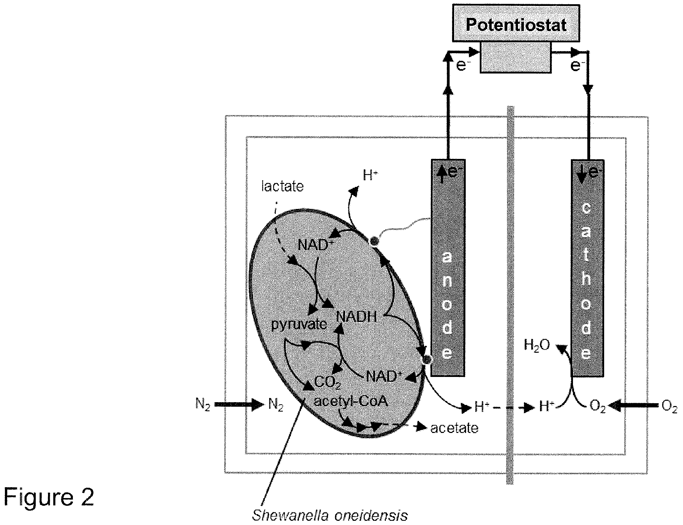

Figure 2 shows a schematic drawing of a bio-electrochemical cell. The electrons released during oxidation are transferred to NAD⁺ to produce NADH, which is reoxidized at the cytoplasmic membrane. From there the electrons are transferred to the outer membrane and the anode, either directly, via nanowires and/or by electron shuttles. The electrons are then transported through the potentiostat to the cathode where O₂ is reduced to produce H₂O.

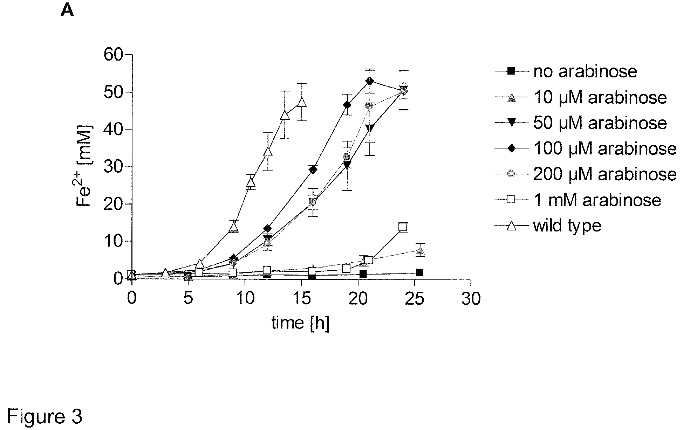

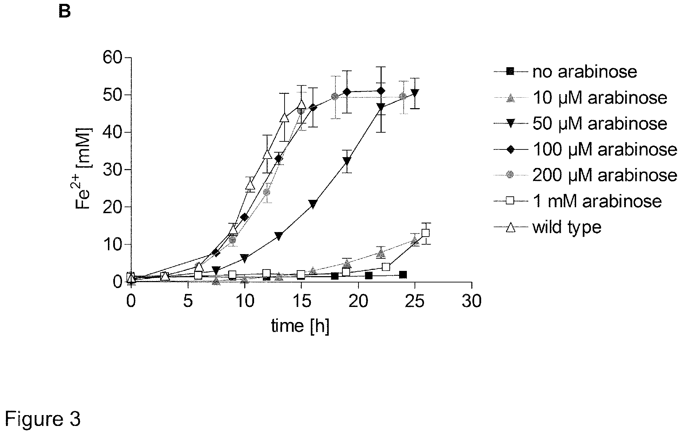

Figure 3A shows the reduction of Fe(III) mediated by plasmid encoded *mtrC* (ΔOMC + pBad *mtrC*ₛₜᵣₑₚ) in comparison to *Shewanella oneidensis* wildtype (wt). Figure 3B shows the reduction of Fe(III) mediated by plasmid encoded *mtrF* (ΔOMC + pBad *mtrF*ₛₜᵣₑₚ). *mtrC* and *mtrF* are each expressed in S. *oneidensis* cells lacking endogenous outer membrane cytochrome (OMC) proteins and under an arabinose inducible promoter such that the rate of Fe(III) reduction directly corresponds to the arabinose concentration.

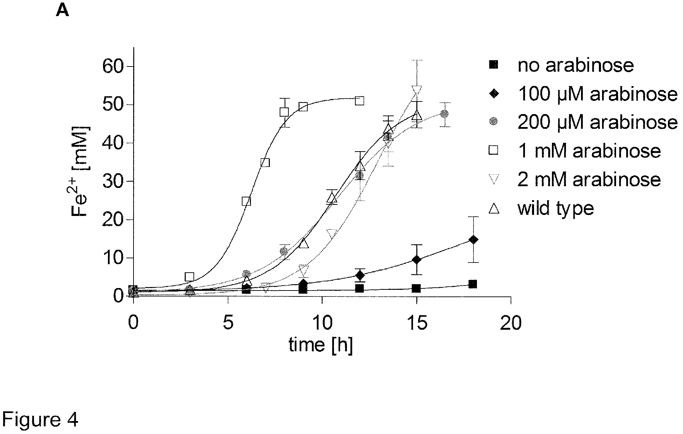

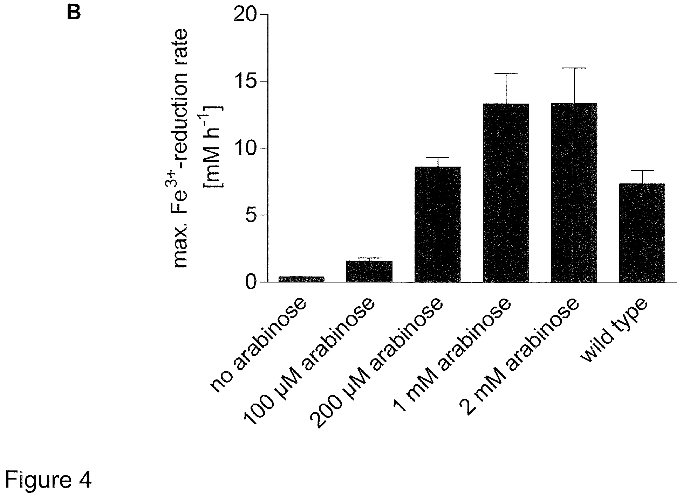

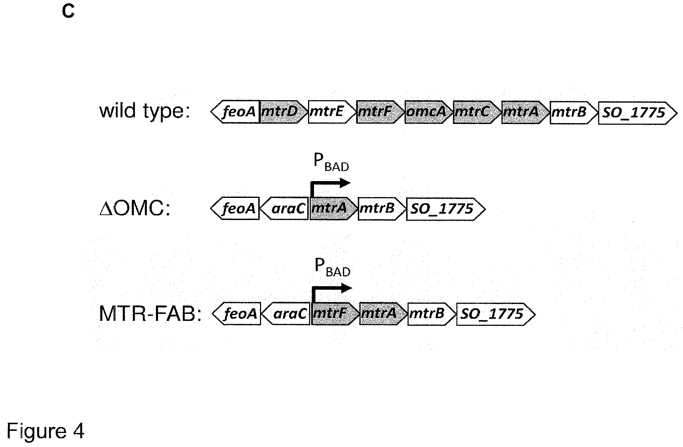

Figure 4A shows the reduction of Fe(III) by the strain S. *oneidensis* MTR-FAB JG410, which contains an arabinose inducible promoter that controls the expression of *mtrA*, *mtrB* and *mtrF*. The ability and efficiency of the cells to reduce Fe(III) was dependent on the concentration of arabinose present in the culture (Figure 4 A). Figure 4B shows the maximum reduction rates achieved for each arabinose concentration. Figure 4 C shows a schematic view of the nucleic acid cluster of S. *oneidensis* MR-1 and mutant strains. The genes coding for *c*-type cytochrome proteins are depicted in grey. The gene *araC* codes for the repressor/activator protein AraC that interacts with the P_{BAD} promoter, which is symbolized in black.

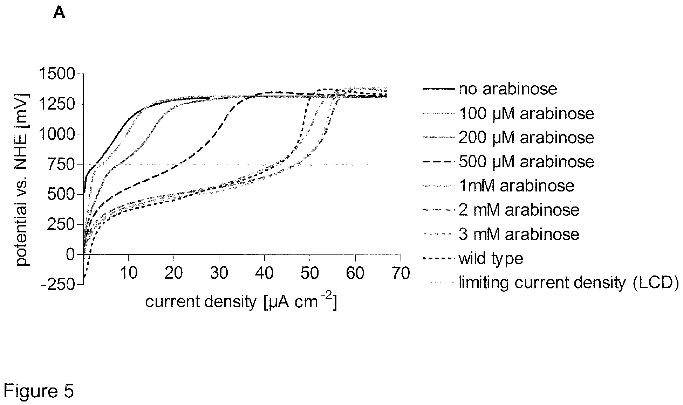

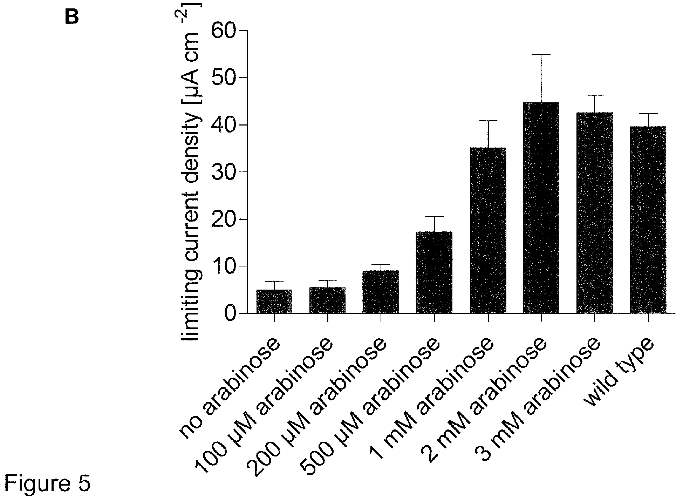

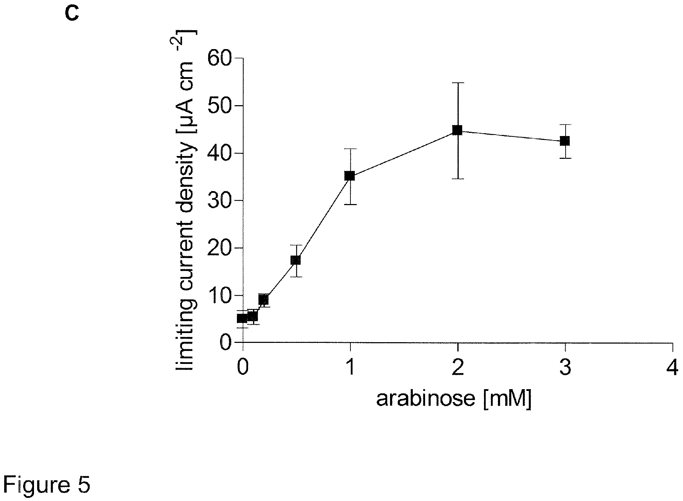

Figure 5A shows a current-ramp-chronopotentiogram of the strain S. *oneidensis* MTR_FAB cultured in the presence of different concentrations of arabinose. The potential was measured versus a standard calomel electrode (SCE) while the current density (µA/cm² anode surface) was constantly increased. Potentials measured against SCE were always calculated to potentials against normal hydrogen electrode (NHE) at the end of the experiment. Figure 5B and C show the limiting current density of the strain *S*. *oneidensis* MTR-FAB cultured in the presence of different concentrations of arabinose. The results are shown for each arabinose concentration as a bar chart (Figure 5 B) and as a graph (Figure 5 C). The limiting current density is defined as the current density at which the cells could no longer transfer sufficient electrons which results in a steep rise of the potential at +750 mV versus NHE.

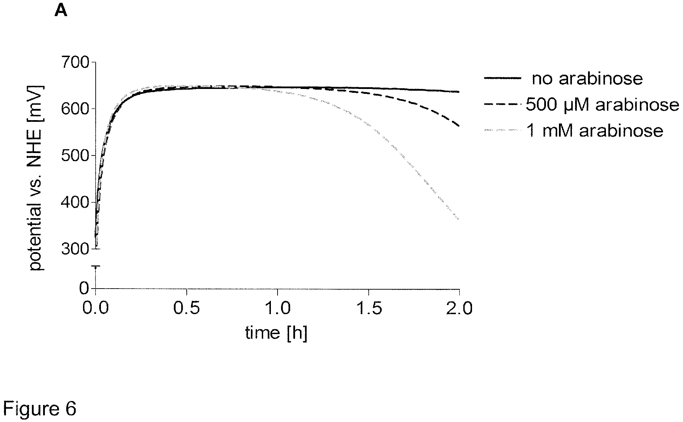

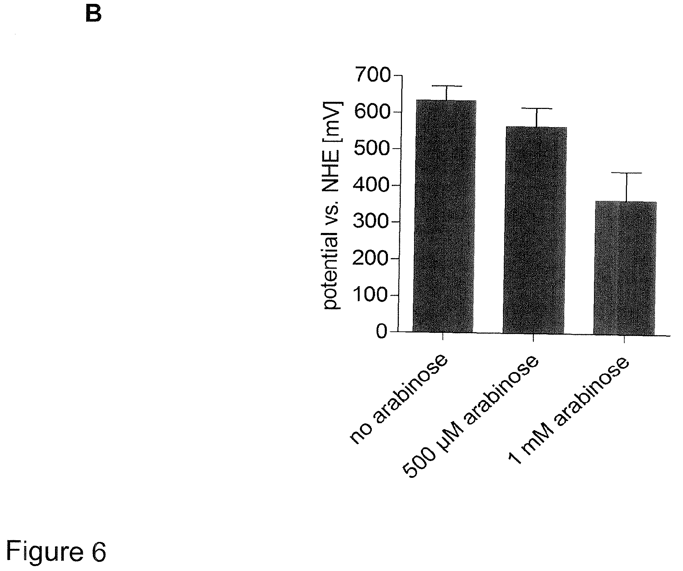

Figure 6 shows the development of the potential during a fast conditioning period.
Figure 6A shows the development of the anode potentials for three different arabinose induction levels. The lines represent mean values of the experiments. Figure 6B shows the potential that was reached two hours after starting the experiment. The data were calculated from duplicate experiments. Error bars represent deviations from the mean.

### Detailed description of the invention

In a first aspect, the description is directed to a nucleic acid construct for detecting a compound comprising a coding region and a regulatory element, which controls expression of the coding region depending on the compound, wherein the coding region comprises the genes *mtrA* or *mtrD;* and *mtrB* or *mtrE;* and *mtrC* or *mtrF.*

Most organisms transfer electrons, which are produced during cellular respiration, to oxygen or other soluble electron acceptors. However, some microorganisms, including dissimilatory metal reducing bacteria, were found to have the ability to use insoluble electron acceptors by transporting electrons to the outside of their outer membrane where the electrons are then transferred to the terminal electron acceptor as e.g. ferric or manganese oxides. Such microorganisms are also able to use an electrode as a terminal electron acceptor without the addition of an artificial mediator, an ability, which is technically employed in microbial fuel cells (MFCs). In MFCs, dissimilatory metal reducing bacteria are cultivated in the presence of an anode such that they release the electrons produced by cell respiration to the electrode system. The electrons flow from the anode through an external circuit to a counter electrode (cathode) and thus create current (Logan and Megan, 2006).

Microorganisms having the ability to transfer electrons to an extracellular acceptor are characterized in that they express proteins transporting electrons from the cytoplasmic membrane, the site of cellular respiration, through the periplasma and the outer membrane to the extracellular side (Figure 1). These proteins, including MtrA, MtrB, MtrC, MtrD, MtrE, MtrF, OmcA and CymA (Figure 1), prolong the respiratory chain from the cytoplasmic membrane up to the extracellular space. The *mtr* genes can be subdivided into three groups each comprising two proteins having corresponding functions: (1) MtrA and MtrD are c-type cytochromes containing heme groups localized in the periplasm, which presumably transport electrons through the periplasm to the outer membrane; (2) MtrB and MtrE, which do not belong to c-type cytochromes, are believed to be transmembrane proteins, mediating the proper localisation of outer membrane cytochromes; (3) MtrC and MtrF are outer membrane cytochromes mediating the transfer of electrons at the outside of the outer membrane to extracellular electron acceptors. Interestingly, MtrA,MtrB and MtrC, as well as MtrD, MtrE and MtrF have the ability to form a complex, respectively. Likewise, the genes *mtrA*, *mtrB* and *mtrC* are organized in one gene cluster and *mtrD*, *mtrE* and *mtrF* in another gene cluster (Figure 4C). Despite that in nature only the complexes MtrA/MtrB/MtrC and MtrD/MtrE/MtrF were so far observed, the proteins of each group were found to have overlapping specificities and activities, resulting in the formation of the complexes such as MtrA/MtrB/MtrF, MtrD/MtrE/MtrC, MtrA/MtrE/MtrC, MtrA/MtrE/MtrF, MtrD/MtrB/MtrC, and MtrD/MtrB/MtrF.

A recombinant *E.Coli* strain expressing a gene construct comprising *mtrC, mtrA* and *mtrB* (*mtrCAB*) genes derived from *S*. *oneidensis* has been described (Jensen et al., 2010) The investigation of several *mtrA*mtrB, *mtrC*paralogs with respect to their functionality and ability to interact with each other and form an active electron transfer chain has been described (Coursolle and Gralni k, 2012).
A microbial fuel cell comprising microorganisms, which express one or more *mtr*genes has also been described (WO 2012/168743 A2).

The inventors used the genes coding for the proteins of the extended respiratory chain to provide a biosensor for detecting compounds. They developed a nucleic acid construct comprising one gene of each of the following groups: (1) consisting of *mtrA* and *mtrD*, and (2) consisting of *mtrB* or *mtrE*, and (3) consisting of *mtrC* and *mtrF* and cloned these genes under a genetic regulatory element, which is regulated by the compound to be detected. Thus, the expression of the *mtr* genes from the construct depends on the presence, absence and/or concentration of the compound. Since the proteins expressed from these genes mediate the cell's ability and efficiency to reduce extracellular electron acceptors, a cell transformed with the construct will transfer electrons to an anode depending on the compound. By culturing the transformed cells in the presence of an anode and connecting the anode to an electric circuit, the produced current indicates the presence, absence and/or concentration of the compound. Thus, the construct of the invention is suitable to provide an electrical biosensor for detecting a given compound.
Although any of complexes formed by *mtr* genes is suitable to carry out the invention, the inventors found that MtrA and MtrB together with MtrF most reliably mediated the generation of electricity depending on the activity of the construct. In particular, complexes comprising MtrA, MtrB and MtrC or MtrF show different electron transfer performances (Figure 3). Whereas MtrC appears to predominate in nature, MtrF was found to be superior to MtrC with respect to biosensor applications. MtrF can be artificially expressed in higher concentrations than MtrC without causing a delayed electron transfer. This is advantageous, since a biosensor should promptly indicate the presence, absence or change in the concentration of a compound to be detected. In addition, the inventors found that by using MtrF, a reliable production of electricity is produced even at comparatively low levels of *mtrF* expression. This makes the construct particularly sensitive. Using mutants of S. *oneidensis* lacking endogenous outer membrane cytochrome (OMC) proteins, the inventors found that expressing MtrF could compensate for the lack of these proteins. Nevertheless, although the combination of *mtrA*, *mtrB* and *mtrF was* found to be more advantageous, also a construct comprising *mtrA*, *mtrB* and *mtrC*, or *mtrD*, *mtrE* and *mtrC*, or *mtrD*, *mtrE* and *mtrF*, or *mtrA*, *mtrE* and *mtrC*, or *mtrA*, *mtrE* and *mtrF*, or *mtrD*, *mtrB* and *mtrC*, or *mtrD*, *mtrB* and *mtrF,* is suitable for detecting a compound.

After revealing their specific properties the *mtr* genes were cloned under a regulatory element, which is directly controlled by a particular compound, thereby providing a sensitive and reliable biosensor. The construct may be adapted for any compound of interest by selecting a regulatory element, which is controlled by the respective compound, and placing the coding region comprising the *mtr* genes under the control of the selected regulatory element. Upon transforming a cell with the resulting nucleic acid construct, the cell will express MtrA or MtrD; and MtrB or MtrE; and MtrC or MtrF. Consequently, the cell will produce a current in an electric circuit depending on the presence, absence and/or concentration of the compound.

In a preferred embodiment, the compound is selected from the group consisting of a hydrocarbon, a peptide, a carbohydrate, an ammonium compound, a carboxylic acid and a salt thereof. In a particular preferred embodiment, the compound is a microbial metabolite. In nature, microorganisms often tightly regulate their gene expression depending on their environment. In particular, they adapt the expression of specific genes according to temperature, pH, and supply of nutrients, oxygen or distinct elements, which they need for their metabolism. Besides detecting the respective substances directly, e.g. through chemotaxis, many microorganisms adapt to their environment through varying their gene expression according to their state of metabolism. Depending on the nutrients available, for example, microorganisms accumulate different metabolites. To regulate gene expression accordingly, many microbial genes are under control of genetic elements, which are regulated by intrinsic metabolites. For example, *Salmonella enterica* catabolizes propionate to pyruvate in the 2-methylcitrate (2-MC) cycle. The enzymes of the 2-MC cycle are expressed under a regulatory element not controlled by propionate directly but by a metabolite of the cycle, namely 2-MC. Other microbial regulatory elements, however, are directly controlled by an external substrate as e.g. the arabinose dependent P_{BAD} promoter. Thus, by using a specific regulatory element, the presence and/or quantity of a specific compound in the environment of the microorganism is detected. In addition, metabolite dependent expression of *mtr* genes can also indicate the living condition of a microbial culture, since the accumulation of specific metabolites is indicative for its stability. This is particularly important for biotechnological applications of microbial cultures e.g. for producing recombinant proteins or generating power. If the ecological system of such cultures is substantially disturbed, the process has to be stopped and the microbial culture replaced, which causes additional expenses and loss of earnings. In biogas production, where various different kinds of microorganisms are cultivated together, the metabolites of one microorganism influence the viability and productivity of others, with some microorganisms even depending on the metabolites of co-cultured organisms (so-called syntrophic microorganisms). For example, during biogas production, fatty acid oxidizing bacteria, as e.g. *Syntrophomonas, Propionibacter, Pelobacter* or *Clostridium,* degrade long chain fatty acid into C₁- and C₂-compounds (acetogenesis), which are then taken up by methanogenic archaea to produce methane. Therefore, high concentrations of long chain fatty acids in biogas cultures often indicate an impaired growth of fatty acid oxidizing bacteria. In addition, they also signify a reduced growth of archaea, because these are inhibited by high concentration of fatty acids in their environment. In contrast, the presence of short chain acids (particularly acetic acid), indicates a functional acetogenesis and provides a good basis for an efficient methanogenesis. Besides acids, also other compounds are indicative for the stability and efficiency of biogas fermentation, as for example the presence and concentration of ammonium compounds as well as the composition of carbohydrates with which the microorganisms are provided. High levels of ammonia are usually produced during decomposition of protein and fat containing material, which is much more abundant than purely hydrocarbon containing compostable waste. Unfortunately, most microorganisms are sensitive to high levels of NH₄⁺ or NH₃ such that the overall efficiency of the biogas fermentation can be significantly impaired by feeding too much protein and fat containing material into the biogas fermenter. Besides the production of ammonia, the fermentation of protein and fat containing material also leads to high levels of acids in the fermenter. Therefore, in this situation, simply measuring the pH will not indicate the instability of the culture, whereas a specific detection of metabolites, as e.g. different acids and/or NH₄⁺ will give detailed information on the state of the fermentation process. For the efficiency of biogas production also the carbohydrate composition within the fermenter is important. For example, monosaccharides are easy to metabolize, whereas polysaccharides need to be hydrolyzed, which is particularly elaborate in case of cellulose and hemicellulose, which contains *inter alia* lignocellulose, xylose, glucose, arabinose and galactose. High concentrations of monosaccharides can cause high levels of acids due to their fast and easy enzymatic decomposition. This can lead to hyperacidity reducing the viability and productivity of microorganisms. High concentrations of complex carbohydrates, in contrast, cause reduced biogas production, since these materials are particularly slowly decomposed, providing insufficient supply for acido- and acetogenesis.

In a particularly preferred embodiment the carbohydrate is arabinose. Since arabinose is a component of hemicellulose it can be used as an indicator for high levels of slowly decomposing material in the fermenter.

In a particularly preferred embodiment, the carboxylic acid is acetic acid or propionic acid. Acetic acid indicates a stable fermentation process and an efficient biogas production, because it is further processed by methanogenic microorganisms to produce methane. Propionic acid, in contrast, is indicative for an inefficient fermentation, since it is the most abundant of long chain fatty acids, which cannot be processed into methane. Both acids decrease the pH of the entire culture, but show specific, even opposite, effects on the fermentation process. Moreover, it was found that the specific ratio of acetic acid and propionic acid is significant for the stability of the fermentation process. Therefore, for monitoring biogas production, two constructs may be used, one expressing the *mtr* genes under an acetic acid dependent regulatory element and one expressing the *mtr* genes under a propionic acid dependent regulatory element.

In a preferred embodiment, the regulatory element comprises a promoter, an inducible promoter, a repressor, an activator and/or an enhancer. The term "promoter" as used herein refers to non-coding nucleic acids which are located upstream of coding regions and are recognized by RNA polymerase enzymes. They mark the site of initiation of transcription and are essential for the expression from the coding region. Promoters directly control transcription and thus protein production. The term "inducible promoter" as used herein, refers to a promoter, which enables transcription dependent on the presence or absence of a specific compound. The compound usually interacts with the promoter, either directly or via a protein, which binds to the promoter. Depending on the binding of the compound, the overall conformation of the promoter region is altered, either enclosing or exposing the RNA polymerase binding site / the site of initiation of transcription. The term "repressor" as used herein refers to a nucleic acid sequence, which is located near the site of initiation of transcription and is recognized and bound by compounds, e.g. proteins. When the repressor is bound by its respective compound, binding of the RNA polymerase enzyme is prevented and transcription of the coding region inhibited. The term "activator" as used herein, refers to a nucleic acid sequence, which is located near the site of initiation of transcription and can be bound by a compound, e.g. a protein. When bound by the respective compound, the activator permits binding of the RNA polymerase enzyme and transcription from the coding region. The term "enhancer" as used herein, refers to short nucleic acid sequences, usually of about 70 base pairs, which support recognition and binding of the RNA polymerase enzyme to the transcription initiation site. Thus, the enhancer promotes onset, level and efficiency of expression from the coding region.

In a preferred embodiment, the regulatory element comprises an arabinose dependent regulatory element, preferably a P_{BAD} promoter. The P_{BAD} promoter activates the expression from the coding region in the presence of arabinose (Guzman et al., 1995). In the absence of arabinose, the protein AraC is bound to the P_{BAD} promoter, thereby masking the site of initiation of transcription. Upon binding of arabinose, AraC is released from the P_{BAD} promoter and transcription is initiated. In addition, the level of gene expression depends on the level of arabinose present. In case, the cell comprising the construct of the invention lacks *araC*, the construct may further include the *araC* gene under a constitutive promoter.

In an alternative preferred embodiment, the regulatory element comprises an ammonium-dependent regulatory element, preferably an amo operon promoter. The pC1 promoter of the ammonia monooxygenase encoding gene cluster in *Nitrosococcus oceani* is only active in the presence of ammonia (EI Sheikh and Klotz, 2008). Putting the coding region comprising the *mtr* genes under this regulatory element, the proteins will only be expressed, and electricity generated, in the presence of and depending on the concentration of ammonia.

In an alternative preferred embodiment, the regulatory element comprises an acetate-dependent regulatory element, preferably a glnAp2 promoter element. The glnAp2 promoter element is derived from *E. coli* and has been adapted to induce gene expression specifically in response to acetate (Farmer and Liao, 2001). Thus, electricity generated from cells comprising the construct of the invention with a glnAp2 promoter element allows the detection of acetate by measuring the electricity generated from these cells.

In an alternative preferred embodiment, the regulatory element comprises a propionate-dependent regulatory element, preferably a P_{prpB} promoter element. The P_{prpB} promoter element is derived from the prpBCDE gene cluster in *E*. *coli* (Lee and Keasling, 2005). The promoter comprises an enhancer-like element which is bound by the propionate derivative 2-methylcitrate (2-MC) and which enables expression from the coding region in the presence of this metabolite. In case the cells comprising the construct of the invention lack enzymes for forming 2-MC, the genes prpEC may be further included into the construct under a constitutive promoter.

In a preferred embodiment, the coding region comprises further genes of the extended respiratory chain, preferably *cymA,* and/or *fccA*, which both encode for proteins transporting electrons from the cytoplasmic membrane through the periplasma to the outer membrane,. The additional expression of these genes enhances the electron transport to the outer membrane, whereas the actual transfer to the anode is still solely performed by MtrC or MtrF. In particular when using the nucleic acid of the invention for transforming cells, which do not have the ability to transfer electrons to an extracellular acceptor by nature, introducing further genes of the extended respiratory chain improves the cells' electron transfer performance.

In a preferred embodiment the regulatory element and the coding region form an operon. An operon is the naturally occurring organization of microbial DNA, in which several genes are expressed from one common promoter. This allows a constant and even expression of several genes e.g. *mtrA*, *mtrB* and *mtrC* or *mtrA*, *mtrB* and *mtrF.*

In a further aspect, the invention is directed to a plasmid comprising a nucleic acid according to the invention. By means of a plasmid, the construct can be easily introduced into any cell. The type of plasmid as well as the further elements, which may be contained in the plasmid, can be selected specifically with respect to the cell to be transformed.

In a further aspect, the description is directed to a cell comprising a nucleic acid according to the invention. By transforming a cell with the construct according to the invention, the cell is enabled to transfer electrons to an extracellular acceptor, in particular to an anode, depending on the presence of a specific compound. Thus, if cultured in the presence of an electrode system, the cell will transfer electrons to the anode and thus generate electricity dependent on the presence, absence and/or concentration of the compound. By measuring the generated electricity with an appropriate device, the presence, absence and/or concentration of the compound can be assessed.

In a preferred embodiment the cell is a microbial cell, preferably a bacterial cell, more preferred a facultative anaerobic bacterial cell. The term "microbial cell" as used herein, refers to single- and multi-cell microscopic organisms including bacteria and archaea. Bacteria are particularly preferred, because they are easy to cultivate having a rather high tolerance for temperature, pH and nutrients fluctuation. Thus, they are suitable to detect compounds in various samples, as e.g. samples from biogas fermentation, which differ in their composition depending on the feeding of the fermenter. Further preferred, the microbial cell is a non-pathogen microbial cell. This allows an uncomplicated but still secure handling of the cell culture such that the detection of the compound can be carried out locally, i.e. at the site of biogas production. The cells may be later collected and professionally exterminated, e.g. by autoclaving. For measurements, however, no laboratory conditions are needed. In anaerobic conditions, the electron transfer to an extracellular acceptor is most efficient, because no other, especially intracellular, electron acceptor is available. In particular, when using facultative anaerobes, cultures should be carried out under anaerobic conditions because oxygen is the most powerful and therefore most preferred electron acceptor.

In a preferred embodiment, the bacterial cell is a *Escherichia coli* cell, a *Shewanella* cell, preferably a *Shewanella oneidensis* cell or a *Geobacter* cell, preferably a *Geobacter sulfurreducens* cell. *E*. *coli* is preferred, since it is a non-infectious and easy to cultivate facultative anaerobe microorganism, which is particularly tolerant to different culture conditions. Likewise, *S*. *oneidensis* is a non-infectious, facultative anaerobe bacteria, which is related to *E. coli* and has the natural ability to reduce extracellular ferric and manganese oxides. Thus, this bacterium expresses proteins of the extended respiratory chain providing an efficient transport of electrons, in particular from the cytoplasmic membrane to the outer membrane. *Geobacter* bacteria, in particular *Geobacter sulfurreducens,* are anaerobe bacteria having the natural ability to transfer electrons through the outer membrane to extracellular electron acceptors. In a preferred embodiment, the cell is devoid of endogenous outer membrane associated cytochrome genes. Lacking endogenous outer membrane cytochromes or cytochrome complexes, any electron transfer to the anode has to be accomplished via MtrC or MtrF, respectively. In this case the transfer of electrons and the production of electricity are dependent on the expression of *mtrC* or *mtrF*, respectively, which in turn is directly controlled by the compound to be detected.

In a further aspect, the invention is directed to a method for detecting a compound in a sample comprising
- providing a microorganism having a nucleic acid construct comprising the genes *mtrA*, *mtrB* and *mtrF* under a regulatory element depending on the compound, the microorganism being devoid of endogenous outer membrane associates cytochrome genes,
- contacting the microorganism with an electrode system such that the microorganism transfers electrons to the anode of the electrode system if the genes are expressed,
- adding a sample to the microorganism, and
- measuring the electron transfer to the electrode system,
wherein the electron transfer to the electrode system indicates the absence, presence and/or concentration of the compound in the sample.

The term "microorganism" as used herein refers to a single- or multi-cell microscopic organism including bacteria and archaea. The term "electrode system" as used herein, refers to a system comprising an anode, a cathode and an external electrical connection. Where applicable, the system may further comprise a device to enable proton transport between the anode and the cathode, e.g. a salt bridge or a proton exchange membrane. The term "sample" as used herein, refers to any portion of a substance, preferably a fluid containing substance, which is to be tested for the presence, absence and/or concentration of a compound, including purely aqueous solutions as well as substances containing solid matter. The sample may be derived from a biotechnological process as e.g. a fermentation process, preferably a biogas fermentation process. The sample may be directly derived or preconditioned, for example filtered, before it is added to the microorganism according to the method of the invention.

For providing a microorganism having a nucleic acid construct comprising the genes *mtrA* or *mtrD*; and *mtrB* or *mtrE*; and *mtrC* or *mtrF*, a nucleic acid, preferably a plasmid, comprising the respective genes, is introduced into a microbial cell. The nucleic acid comprises these genes under a regulatory element, which is controlled by the compound to be detected. After providing the microorganism, a suitable medium is inoculated with the microorganism to prepare a stable culture. The microorganisms are contacted with the electrode system by introducing the anode into the culture medium, such that the microorganisms attach to the anode and form a biofilm covering the same. This process can be supported by applying a low current, e.g. about 200 nA to about 700 nA, preferably about 500 nA, to the electrode system. A tight and stable biofilm on the anode's surface increases the efficiency of the electron transfer from the microorganisms to the anode. The process can be further supported, but does not have to be supported by adding low concentrations of the compound to the media, e.g. about 5 µM to about 50 µM, preferably about 5 µM to about 20 µM, more preferred about 10 µM. Low levels of the compound induce the expression of *mtr* genes from the construct and thus enable the cells to transfer electrons to the anode. The transfer of electrons to the anode was found to improve biofilm formation. In addition, in the presence of low *mtr* gene expression and low voltage, the formation of the biofilm causes a continuous reduction of the anode potential, which stops once the biofilm is fully established. At this point, the microorganisms are most suitable for performing the further steps of the method.

For testing a given sample for a compound, the sample is added to the microorganisms. This may be achieved either by introducing the sample into the microorganisms' culture or by transferring the anode covered by the microorganism biofilm into the sample. The latter is particularly preferred if the sample itself is derived from a microbial process, such that the sample provides a suitable environment for the microorganisms. In case the sample is not suitable to support the microorganisms or is expected to include substances, which could inhibit growth and viability of the microorganisms, it is preferred to add the sample into the microorganisms' culture. Depending on the kind of regulatory element controlling the expression from the coding region (e.g. repressor, activator and/or enhancer), the microorganisms will express the *mtr* genes depending whether the compound interacting with the regulatory element is present in the sample. For example, if the expression of *mtrA*, *mtrB* and *mtrF* is induced by the interaction of the compound with the regulatory element, the microorganisms will produce the respective proteins and establish an extended respiratory chain. Accordingly, the microorganisms will transfer electrons to the anode, if the compound is present in the sample. The amount of electrons transferred depends on the amount of MtrA, MtrB and MtrF protein, which in turn is determined by the concentration of the compound present in the sample. The amount of electrons transferred from the microorganisms to the anode can be determined by measuring the current in the electrode system. Interestingly, the inventors found a linear relation between the concentration of the compound and the maximum electron transfer capacity of the microorganisms. The maximum electron transfer capacity can be expressed as the limiting current density of the microorganisms. To determine this, the inventors increased the amperage step wise and measured the voltage at the anode supported by the microorganisms' electron supply. The amperage was normalized to the surface area of the anode to obtain the current density (µA/cm²). Plotting the current density against the measured potential revealed a phase of linear increase flanked by two phases of rather exponential potential increase (Figure 5 A). The inventors found that as long as the microorganisms can produce and transfer sufficient electrons to the anode to support the applied amperage (linear phase), the microorganisms behave like an Ohmic resistance. If the amperage is further increased, the microorganisms are no longer able to support the current flow and the anode potential rises steeply. This point indicates the maximum electron transfer capacity of the microorganisms. To allow comparison of different strains, the current density which was reached at a potential of +750 mV versus NHE was defined to be the limiting current density of a bacterial strain. An anode potential beyond +750 mV represents a potential at which electrochemical processes like hydrolysis become predominant. The inventors further found that the limiting current density is directly depending on the expression level of *mtr* genes leading to a linear relation between the limiting current density and the concentration of the compound (Figure 5 C). Thus, if the microorganisms are cultured in the presence of a compound activating expression from the coding region, higher levels of the compound cause an increased maximum electron transfer capacity and limiting current density. This is because higher levels of Mtr proteins enable the microorganisms to transfer more electrons per time and surface area to the electrode and thus to support a higher current flow to the electrode system.

By determining the limiting current density of microorganisms cultured at various concentrations of the compound, a characteristic line representing the relation between the maximum electron transfer capacity and the concentration of the compound can be established. Afterwards, the concentration of the compound in an unknown sample can be assessed by identifying the limiting current density of the microorganisms in the presence of the sample using the characteristic linear relationship between the compound's concentration and the limiting current density.

Thus, the method allows the direct detection of a compound and the determination of its concentration in a sample via an electric device. In addition, the method does not require any elaborate technical equipment and does not involve complex calculations.

In a preferred embodiment, the sample is a sample of a microbiological process, preferably a fermentation process, more preferred a biogas fermentation process. In general, any sample may be tested for a given compound using the method of the invention. However, the method is particularly preferred to test samples derived from microbiological processes, since these samples usually provide an environment which is suitable to support the microorganisms used in the method. Moreover, the microorganism used in the method may be specifically selected according to the sample to be tested. For example, the sample can be provided in a suitable chamber and the anode covered by the biofilm of microorganisms can be directly introduced into the chamber, such that the sample can be tested immediately where the microbial process is carried out. This is in particular advantageous for biogas fermentation processes, which require an often or even continuous control of the fermentation process due to the simultaneous cultivation of very different microorganisms.

In a preferred embodiment, the anode comprises a noble metal or carbon. In contrast to anodes made from other metals as e.g. ferric iron, anodes comprising a noble metal or carbon do not corrode. In addition, they are far more efficient in transferring electrons. Since electrodes comprising noble metals as e.g. gold or platin are expensive, carbon containing electrodes are particularly preferred. In addition, carbon electrodes can be provided in various designs having large surface areas to support biofilm formation. Carbon electrodes include those comprising carbon cloth, carbon paper and graphite rods, plates or granules, as well as reticulated vitreous carbon (RVC).

In a preferred embodiment, the microorganism transfers the electrons by direct contact between MtrC or MtrF, respectively, and the anode or by a soluble electron shuttle. The proteins MtrC and MtrF were found to be able to mediate electron transfer to a terminal electron acceptor at the extracellular side of the outer membrane either by direct contact or via soluble electron shuttles. Direct contact to the anode can be established by attachment of the entire bacterial cell or by bacterial nanowires. Nanowires are long and thin extensions of the outer membrane that can bridge rather long distances to contact the electron acceptor. Alternatively, MtrC and MtrF are both able to transfer electrons to soluble shuttles, which then diffuse and transfer the electron to the terminal electron acceptor. A soluble electron acceptor, as e.g. humine substances and flavin, may be added to the culture.

In a preferred embodiment, the electron transfer in the electrode system is measured using a potentiometric or an amperometric method. For determining the limiting current density of the microorganisms, the current is continuously increased over a defined current range. The current at which a characteristic potential is reached is defined as the limiting current of which the microorganism's limiting current density can be calculated. This is preferably done using a potentiometric device and a standard calomel electrode (SCE).

In a further aspect, the invention is directed to a biosensor for detecting a compound comprising
- an electrode system,
- a device for measuring electron transfer in the electrode system, and
- a microorganism having a nucleic acid construct comprising the genes *mtrA, mtrB* and *mtrF,* which transfers electrons to the electrode system if the genes are expressed,
wherein the regulatory element is controlled by the compound and the microorganism is devoid of endogenous outer membrane associates cytochrome genes.

Upon exposing the biosensor to a sample to be tested, the microorganisms express the *mtr* genes depending on the presence of a compound, which controls the regulatory element. Since the capacity of the microorganisms to transfer electrons to the anode is directly dependent on the amount of Mtr protein generated in the cells, the concentration of the compound can be determined from the electron transfer capacity of the microorganisms. The biosensor measures the electron transfer capacity of the microorganisms when cultured in the presence of a sample such that the concentration of the compound in the sample can be deduced.

In a preferred embodiment, the microorganism is selected from the group consisting of *Escherichia,* preferably *Escherichia coli, Shewanella,* preferably *Shewanella oneidensis*, and *Geobacter,* preferably *Geobacter sulfurreducens.*

In a preferred embodiment, the microorganism is devoid of endogenous outer membrane associated cytochrome genes. In a particular preferred embodiment, the microorganism is *Shewanella oneidensis MR-1^{ΔOMC}* (Bücking et al., 2010). In general, S. *oneidensis* is preferred for the biosensor, since it has the natural capacity to transfer electrons over the outer membrane and therefore also comprises the proteins, which efficiently transport electrons from the cytoplasmic membrane to the outer membrane (e.g. CymA and fumarate reductase). *S*. *oneidensis MR-1^{ΔOMC}*, although harbouring the other proteins of the extended respiratory chain, lacks any endogenous outer membrane cytochromes, i.e. endogenous *mtrF*, *mtrC*, OmcA, SO_1659 and SO_2931. Therefore, upon introducing a nucleic acid construct comprising *mtrC* or *mtrF*, any electron transfer through the outer membrane is mediated by *mtrC* or *mtrF* expressed from the artificial construct. A specific S. *oneidensis* mutant was established by introducing the genes encoding *mtrF*, *mtrA* and *mtrB* placed in a row behind a P_{BAD}-promoter *(pBADmtrFAB)* in the genome of a ΔOMC strain backbone. This resulted in the *S*. *oneidensis* strain MTR-FAB JG410 (*ΔOMCpBADmtrFAB*) (deposited at the Leibniz-Institut DSMZ-Deutsche Sammlung von Mikroorganismen und Zellkulturen GmbH, Germany on July 25, 2012, under DSM 26201). MtrA, MtrB and MtrF are all expressed from the same operon (Figure 4 C) and therefore increase in equal measure proportionally with induction of expression by arabinose.

In a preferred embodiment, the anode of the electrode system comprises a noble metal or carbon.

In a further aspect, the invention is directed to a biosensor for monitoring a fermentation process comprising at least one bio-electrochemical cell with a culture of a microorganism having a nucleic acid construct comprising the genes *mtrA*, *mtrB* and *mtrF* under a regulatory element such that the microorganism transfers electrons to an electrode of the bio-electrochemical cell if the genes are expressed, wherein the expression of the genes depends on a product or a substrate of the fermentation process and the microorganism is devoid of endogenous outer membrane associates cytochrome genes.

The term "bio-electrochemical cell" refers to a device to convert biochemical energy into electrical energy. Within the bio-electrochemical cell, microorganisms convert energy bound in biomass, e.g. in carbohydrate, proteins or fatty acids, into electric energy by catabolizing the biomass. The electrons obtained during this process, are used by the microorganism to support their own energy needs and are finally transferred to a suitable electron acceptor. In the bio-electrochemical cell, the terminal electron acceptor is an anode, through which the electrons are introduced in an electrical circuit, e.g. for collecting energy (so called microbial fuel cell (MFC)). Using the bio-electrochemical cell as a biosensor, the capacity of the microorganisms to transfer electrons to the anode is measured and used to determine the presence, absence and/or concentration of a compound. The term "fermentation" as used therein refers to a process conducted by cells, wherein organic substrate is oxidized for obtaining energy and the electrons released thereby are transferred to endogenous organic compounds as electron acceptors. The term "product of a fermentation process" as used herein refers to any compound generated by a cell during a fermentation process including intermediate products of catabolizing organic substances, as well as products resulting from terminal electron transfer. The biosensor comprises a culture of a microorganism having a nucleic acid construct comprising the genes *mtrA* or *mtrD*; and *mtrB* or *mtrE*; and *mtrC* or *mtrF* under a regulatory element, which in turn is controlled by a product of the fermentation process. Since the expression level of the *mtr* genes defines the electron transfer capacity of the microorganism, the biosensor determines the presence, absence and/or concentration of the product of the fermentation process by measuring the electron transfer capacity of the microorganisms. The biosensor may be applied by taking a sample from the fermentation process and introducing the same into the bio-electrochemical cell or by connecting the bio-electrochemical cell directly to the fermentation process such that the bio-electrochemical cell is continuously supplied with a sample of the fermentation process. In the latter embodiment, the biosensor provides a real-time monitoring of the production of a given product of the fermentation process. The biosensor is particularly suitable for controlling complex fermentation processes as e.g. biomass fermentation. Biomass fermentation is used for providing alternative energy resources, in particular methane gas but is also investigated for producing hydrogen (H₂). Since biomass fermentation is a complex and sensitive process based on many different microorganisms it is particularly important to keep the fermentation running stably. Disturbances of the system can lead to substantial reduction of biogas production or even abortion of the fermentation process. In this case, the fermenter has to be emptied and cleaned and a new fermentation process induced. This does not only cause substantial loss of revenue but also undesired expenses.

In a preferred embodiment, the product of the fermentation process is propionic acid or acetic acid. During biogas fermentation, which is based on the coexistence of several different microorganisms, the concentrations of propionic acid and acetic acid, in particular the ratio of both acids, was found to be indicative for the stability of the entire fermentation process. During biomass fermentation, acidogenic bacteria decompose biomass into acids, alcohols and water. The mostly long chain carbon acids (with more than two carbon atoms) are converted to acetic acid, acetate, C₁-compounds and water by acetogenic bacteria. The acetate and the C₁-compounds are further metabolized by methanogenic microorganisms, mostly archaea, to produce methane and CO₂. An accumulation of long chain acids, predominantly propionic acid, and a decrease in acetic acid and acetate indicates a reduced acetogenesis leading to reduced production of biogas. Likewise it indicates impaired conditions for the methanogenic archaea, which are sensitive to acidic environments. Therefore, propionic acid and acetic acid are particular preferred indicators for the condition of a biomass fermentation process. Accordingly, the invention is also directed to a biosensor for monitoring a fermentation process comprising two bio-electrochemical cells each with a culture of a microorganism having a nucleic acid construct comprising the genes *mtrA*, *mtrB* and *mtrF* under a regulatory element, such that the microorganism transfers electrons to an electrode of the bio-electrochemical cell if the genes are expressed, wherein the regulatory element of the first microorganism is propionic acid dependent regulatory element and the regulatory element of the second microorganism is an acetic acid dependent regulatory element wherein the microorganism is devoid of endogenous outer membrane associates cytochrome genes.

In a further aspect, the description is directed to the use of a nucleic acid construct for detecting a compound comprising a coding region and a regulatory element, which controls expression of the coding region depending on the compound, wherein the coding region comprises the genes *mtrA* or *mtrD*; and *mtrB* or *mtrE*; and *mtrC* or *mtrF.* In a further aspect, the invention is directed to the use of a cell for detecting a compound, comprising a nucleic acid construct with a coding region and a regulatory element, which controls expression of the coding region depending on the compound, wherein the coding region comprises the genes *mtrA*, *mtrB* and *mtrF,* wherein the cell is devoid of endogenous outer membrane associates cytochrome genes.

### Examples

### 1. Culture Media

Cultures of aerobic growing microorganisms including *E. coli* and *S*. *oneidensis*, where grown in LB-media containing 1 % (w/v) bactotrypton, 0,5 % (w/v) yeast extract and 0.5 % (w/v) NaCl. For growing S. *oneidensis* under anaerobic conditions, different minimal media were used. Tables 1 and 2 show the contents of PBS (phosphate buffered saline)-buffered media. Table 3 shows the contents of HEPES-buffered ferric citrate-media, which was produced using a stock solution as shown in table 4. In all of these media lactate (50 mM) was used as electron donor as well as carbon source. In some cases fumarate (100 mM) was additionally used to provide an electron acceptor. A stock solution providing trace elements was used as shown in table 5.

**Table 1: PBS-MFC-media (anode chamber of the MFC)**

| Substances | per liter | final concentration |
|---|---|---|
| CaCl₂-solution (0,1 M) | 1 ml | 0,1 mM |
| Caseinhydrolysate | 1 g | 0,1% (w/v) |
| KCI | 0,2 g | 2,7 mM |
| KH₂PO₄ | 0,24 g | 1,76 mM |
| Mg₂SO₄-solution (1 M) | 1 ml | 1 mM |
| NaCl | 8g | 137 mM |
| Na₂HPO₄·2H₂O | 1,78 g | 10 mM |
| (NH₄)₂SO₄ | 1,18 g | 9 mM |
| Na-Lactate 50% (w/w) | 11,2 g | 50 mM |
| 100 X trace elements solution | 10 ml | |

**Table 2: PBS-fumarate-media**

| Substances | per liter | final concentration |
|---|---|---|
| CaCl₂-solution (0,1 M) | 1 ml | 0,1 mM |
| Caseinhydrolysate | 1 g | 0,1% |
| fumaric acid | 11,6 g | 100 mM |
| KCI | 0,2 g | 2,7 mM |
| KH₂PO₄ | 0,24 g | 1,76 mM |
| Mg₂SO₄-solution (1 M) | 1 ml | 1 mM |
| NaCl | 8 g | 137 mM |
| Na₂HPO₄·2H₂O | 1,78 g | 10 mM |
| (NH₄)₂SO₄ | 1,18 g | 9 mM |
| Na-Lactate 50% (w/w) | 11,2 g | 50 mM |
| NaOH | 8 g | 200 mM |
| 100 X trace elements solution | 10 ml | |

**Table 3: HEPES-ferric citrate-media**

| Substances | per liter | final concentration |
|---|---|---|
| CaCl₂-solution (0,1 M) | 1 ml | 0,1 mM |
| Caseinhydrolysate | 1 g | 0,1 % |
| Fe(III)-Citrate | 12,24 g | 50 mM |
| HEPES-ferric-citrat-stock solution (20X) | 50 ml | |
| Mg₂SO₄-solution (1 M) | 1 ml | 1 mM |
| Na-Lactate 50% (w/w) | 11,2 g | 50 mM |

**Table 4: stock solution for HEPES-ferric citrate-media (20x)**

| Substances | per liter | final concentration |
|---|---|---|
| KH₂PO₄ | 1,98 g | 0.73 mM |
| K₂HPO₄ | 4,42 g | 1.27 mM |
| HEPES | 23,8 g | 5 mM |
| (NH₄)₂SO₄ | 22 g | 8.32 mM |
| NaCl | 58,4 g | 50 mM |
| 100 X trace elements solution | 200 ml | |

**Table 5: stock solution for trace elements (100x)**

| Substances | per liter | final concentration |
|---|---|---|
| CoCl₂ | 64,9 mg | 5 µM |
| CuSO₄·5H₂O | 5 mg | 0,2 µM |
| Fe(II)Cl₂·4H₂O | 107,4 mg | 5,4 µM |
| H₃BO₃ | 350 mg | 56,6 µM |
| MnSO₄·H₂O | 22 mg | 1,3 µM |
| Na₂EDTA | 2501,5 mg | 67,2 µM |
| Na₂MoO₄·2H₂O | 94,4 mg | 3,9 µM |
| Na₂SeO₄·6H₂O | 39,5 mg | 1,5 µM |
| NaCl | 58,4 mg | 10 µM |
| NiCl₂·6H₂O | 118,9 mg | 5 µM |
| ZnSO₄·7H₂O | 28,8 m | 1 µM |

*E. coli* were cultured at 37°C and S. *oneidensis* at 30°C, respectively.

For anaerobic ferric-citrate cultures and MFC, pre-cultures were prepared in 100 ml PBS-fumarate-medium and subsequently inoculated with a LB-pre-culture. After the microorganisms reached the exponential growth phase, the cultures were centrifuged and washed twice in PBS-buffer. The microorganisms, which were to be used for ferric iron reduction experiments, were dissolved in PBS-buffer at an optical density (OD)₆₀₀ of 0.4. This pre-culture was used to inoculate HEPES-ferric-citrate media, which was furnished with arabinose, where appropriate. Microorganisms, which were to be used for MCF experiments, were dissolved in PBS-anode-medium containing arabinose, where appropriate. The final OD₆₀₀ of the the anode compartment was adjusted to 0.4 by addition of a concentrated pre-culture suspension.

### 2. Preparation of S. oneidensis deficient of outer membrane cytochromes

A S. *oneidensis* mutant lacking endogenous outer membrane cytochrome genes, i.e. *mtrF*, *mtrC*, *OmcA, SO_1659* and *SO_2931*, was constructed according to Bücking et al. 2010.

### 3. Ferric iron/Fe (III)-reduction assay

For determining Fe(II) concentrations in anaerobic cultures, a ferric iron reduction assay with Ferrozine (Dinatrium-4-[3-pyridin-2-yl-6-(4-sulfonatophenyl)-1,2,4-triazin-5-yl]-benzosulfonat) was used. After inoculating the culture medium with the pre-culture, samples were taken at various time points for determining the Fe(II) concentration. The first sample was taken immediately after inoculation giving the Fe(II) concentration at t = 0. Further samples were taken at least each three hours.

The results of the Ferrozine assay were analyzed using the GraphPadPrism-software (GraphPad Inc., La Jolla, USA). For each mutant and each arabinose concentration, the mean Fe(II) concentrations were determined and plotted against the time point each sample was taken. Thereby, the chronological ferric iron reduction for each culture was displayed.

For determining the maximum Fe(III) reduction rate for each mutant and each arabinose concentration three reduction experiments were conducted, respectively. For analysis, the data for each replicate was individually plotted and analyzed according to non-linear regression and sigmoidal dose-response (variable slope) as provided by the GraphPadPrism-software. This allowed reproducing the actual reduction process in high approximation.

### 4. Microbial fuel cell (MFC)

For determining the electron transfer rates of *S*. *oneidensis* a specifically designed MFC was used. The reaction chambers had a volume of 25 ml and were separted by a proton exchange membrane (Fumapem F-950, Quintech, Göttingen, Germany). The same type of membrane was used to separate the anode from the reference electrode chamber. A potentiostat (WaveNow Portable USB Potentiostat, Pine Research Instrumentation, Raleigh, USA) was connected to the electrodes. This potentiostat was also used as a galvanostat, allowing defining the current flow to which the entire system had to adapt. Likewise, the potentiostat was connected to a standard calomel-reference electrode (Sensortechnik Meinsberg, Ziegra-Knobelsdorf, Germany) which was used to determine the anode potential at a predefined amperage. This allowed determining the electron transfer capacity of the microorganisms independently of the cathode at different concentrations of arabinose. The electrode material used was SIGRATHERM graphite felt (SGL Group, Wiesbaden, Germany) with a thickness of 1 mm. It was cut to squares of 1 cm² and fixed onto a titanium grid. The anode chamber was filled with 22.5 ml PBS-MFC-medium, which was furnished with arabinose, where applicable. The cathode and the standard electrode chamber were filled with 25 ml and 4 ml of PBS buffer, respectively. To ensure anaerobic conditions in the anode chamber, it was permanently purged with nitrogen gas. The cathode chamber was purged with compressed air to ensure non-limiting electron flow. For inducing biofilm formation of the microorganisms, the anode chamber was inoculated with the pre-culture and the potentiostat was used to apply 500 nA to the anode for ten hours. Afterwards, the current-ramp-chronopotentiometric analysis was performed with increasing the amperage from 500 nA to 120 µA by steps of 800 pA/sec. Current densities were calculated taking the electrode surface of 1.8 cm² into account (Figure 5). The potentials were measured versus a standard calomel-electrode and recalculated for a normal hydrogen electrode. Thus, the potential of 0 mV versus the standard calomel electrode corresponded to +237.9 mV versus a normal hydrogen electrode.

### 5. Electron transfer by mtrC and mtrF

Previous experiments showed that *S*. *oneidensis* mutants lacking endogenous outer membrane cytochromes (ΔOMC-mutants) supplemented with *mtrC* or *mtrF* showed higher reduction rates compared to mutants supplemented with other outer membrane cytochromes (Bücking et al., 2010). To further investigate the properties of *mtrC* and *mtrF*, ΔOMC-mutants expressing *mtrC* or *mtrF* under an arabinose inducible regulatory element (P_{BAD}-promoter) were analyzed in the presence of different arabinose concentrations. The mutants were supplemented with *mtrC* or *mtrF* by introducing plasmids containing the respective construct under a P_{BAD} promoter. Additionally, the endogenous genes for *mtrA* and *mtrB* were put under the same promoter (Bücking et al., 2010). For investigating the ferric iron reduction capacity of both mutants at different concentrations of arabinose, the mutants were cultured in media furnished with 0 µM, 10 µM, 50 µM, 100 µM, 200 µM and 1 mM arabinose and the Fe(II) production was determined as described. Ferric iron reduction by wild type S. *oneidensis* was used as a reference (Figure 3). These experiments revealed that in the absence of arabinose both mutants were unable to reduce ferric citrate. However, from arabinose concentrations of 10 µM onwards, both mutants showed an increased ferric iron reduction. With increasing arabinose concentration, also the reduction rate increased and the onset of reduction occurred faster (Figures 3A and B). In particular at arabinose concentrations of 100 and 200 µM distinctive differences were apparent between the two mutants. S. *oneidensis* supplemented with *mtrF* (ΔOMC P_{BAD} *mtrF*) showed ferric iron reduction levels comparable to wild type *S*. *oneidensis* (Figure 3 B), whereas *S*. *oneidensis* supplemented with *mtrC* (ΔOMC P_{BAD} *mtrC*) showed a delayed onset and decreased rates of ferric reduction (Figure 3 A). This showed that *mtrF* in comparison to *mtrC* promotes higher rates and a faster onset of electron transfer.

### 6. Cloning S. oneidensis MTR-FAB

*MtrC* is able to form a stable complex with *mtrA* and *mtrB* to transport electrons through the outer membrane. To investigate whether also *mtrF* is able to form a complex for electron transport with *mtrA* and *mtrB*, *mtrA*, *mtrB* and *mtrF* were expressed at similar levels to promote the formation of a 1:1:1-complex. Therefore, a S. *oneidensis* mutant was produced which lacked any outer membrane cytochromes but carried a coding region comprising *mtrA*, *mtrB* and *mtrF* under an inducible promoter.

Since the ΔOMC mutant already contained *mtrA* and *mtrB* under the P_{BAD} -promoter, *mtrF* was introduced into the *mtrA* and *mtrB* containing codon. Cloning was done using the suicide plasmid pMQ150-*mtrFAB*. This plasmid contains the selection marker *kanR* and *sacB* and an origin replication (oriR6K). *mtrF* was cloned into this plasmid using the sections UP and DOWN, which are homolog to 500 bp upstream and downstream of the site of insertion in the genome of S. *oneidensis* ΔOMC, respectively. Since the gene sections for *UP* and *mtrF* were already contained in the P_{BAD} 202/D-TOPO-*mtrF-*plasmid, this was used as a template for PCR with the primers *mtrFAB* UP_for (SEQ ID NO. 1: ATG ATT ACG AAT TCG AGC TCG GTA CCC GGG CTG ACC GCG AAT GGT GAG) and *mtrFAB* STREP_REV (SEQ ID NO. 2: TCT TCA TAA TAG GCT TCC CAA TTT GTC CCA TTA TTT TTC GAA CTG CGG GTG). The DOWN-fragment was produced using the primers *mtrFAB* DOWN_for (SEQ ID NO. 3: TGG GAC AAA TTG GGA AGC C) and *mtrFAB* DOWN_REV (SEQ ID NO. 4: CGG CCA GTG CCA AGC TTG CAT GCC TGC AGG GCA ACA TCG GCA TTG TCA TG). The genome of the S. *oneidensis* ΔOMC-mutant was used as a template. The produced fragments were assembled using a fusion-PCR and the primers *mtrFAB* UP_4 and *mtrFAB* DOWN_REV. The resulting insertion fragment was integrated into the pMQ150 plasmid and the resulting pMQ150-*mtrFAB*-plasmid transformed into *E. coli* S-17. Using *E*. *coli* S-17, the plasmid was conjugated into *S*. *oneidensis ΔOMC* followed by homologous recombination. The resulting mutant *S*. *oneidensis* MTR-FAB JG410 (deposited at the Leibniz-Institut DSMZ-Deutsche Sammlung von Mikroorganismen und Zellkulturen GmbH, Germany on July 27, 2012, under DSM 26201) expressed *mtrF*, *mtrA* and *mtrB* from a common arabinose inducible promoter within the genome (Figure 4 C).

### 7. Ferric iron reduction of S. oneidensis MTR-FAB

The ferric iron reduction capacity of the mutant *S*. *oneidensis* MTR-FAB was investigated at different arabinose concentrations. After inoculating the medium, Fe(II) concentrations were measured at different time points and the maximum reduction rates of the mutant determined for individual concentrations of arabinose (Figure 4).

Regarding the ferric iron reduction process, the mutant *S*. *oneidendsis* MTR-FAB behaved similar to the wild type.

Investigating the ferric iron reduction capacity of this mutant, revealed a reduced maximum reduction rate at low levels of arabinose, whereas at high levels of *mtrA*, *mtrB* and *mtrF* expression, the maximum reduction rate of the mutant was even increased compared to the wild type. Additionally, a faster onset of ferric iron reduction, i.e. electron transfer to the extracellular electron acceptor, was observed for some arabinose concentrations. In summary, *S*. *oneidensis* MTR-FAB showed higher reduction rates and a faster onset of electron transfer, making the mutant advantageous over the wild type *S*. *oneidensis*, in particular for applications in artificial systems.

### 8. Using S. oneidensis MTR-FAB with a MFC

The performance of a MFC is mostly determined by the efficiency of the redox reduction in the anode and cathode chamber. To determine the efficiency of the anode independently of the cathode, a potentiostat was used to determine the potential difference between the bacteria and the anode. This potential difference represents the efficiency of the electron transport from the bacteria to the electrode. A positive anode potential thus represents a significant fall of voltage between the bacteria and the electrode. Likewise, a relatively high potential at constant amperage indicates the limit of the electron flow from the bacteria. In addition, according to Ohm's law, a higher electric resistance causes an increased fall of voltage if the electron flow remains constant. The specific electrical resistance of the bacteria is directly depending on the outer membrane cytochromes, which mediate the electron transfer. Thus, monitoring the potential at different amperages was used to reveal the electron transfer capacity of *S*. *oneidensis* MTR-FAB at different concentrations of arabinose. Starting the experiment, the anode chamber of the MFC was filled with anode media and the cathode chamber with PBS buffer. The anode medium was complemented with arabinose, where and at concentrations appropriate. The MFC was then connected to the potentiostat and the gas supply. The anode chamber was inoculated with a *S*. *oneidensis* MTR-FAB pre-culture derived in PBS-fumarate medium, which also included arabinose, where appropriate. The culture of the bacteria was expanded in the anode chamber for about ten hours. During this time an amperage of 500 nA was applied to the anode for supporting attachment of the bacteria to the anode material.

During this conditioning phase, a continuous decrease of the anode's potential was observed, which finally reached a plateau indicating that the bacteria formed a complete biofilm around the anode. Following the conditioning phase, current-ramp-chronopotentiometric measurements were carried out to determine the electron transfer capacity of *S*. *oneidensis* MTR-FAB at different arabinose concentrations. To do so, the amperage of the potentiostat was increased from 500 nA to 120 µA in steps of 800 pA/sec. For each amperage the potential difference between the anode and the standard electrode was measured. To determine the current density, the amperage was normalized to the surface of the electrode, which was 1.8 cm². The calculated current densities were then plotted against the measured potential. The resulting current-ramp-chronopotentiogram reveals four different phases of the experiment (Figure 5 A). At the beginning of the current sweep, a steep increase in potential was observed followed by a more linear increase in response to high occurrence. In this phase, the bacteria behaved analogous to an ohmic resistance. At higher current fluxes a further rapid increase in potential was observed, after which all curves merged into a common line. The current density at which bacteria failed to provide sufficient quantities of electrons to sustain the given current flux represents a characteristic feature of each mutant strain. To simplify comparison between the performances of the different bacteria cultures, the limiting current density of the bacteria was defined as current flux beyond which the measured anode potential first exceeded +750 mV (limiting potential) versus normal hydrogen electrode. Interestingly, this revealed a linear relation between the limiting current density of each culture and its arabinose concentration (Figure 5 C). The limiting current density increased with the arabinose concentration and reached levels comparable to the limiting current density of the wild type at a concentration of 1 mM arabinose. Further increasing the arabinose concentration increased the limiting current density even above that of wild type S. *oneidensis*. In particular, at low arabinose concentrations (0.1 to 1 mM), thus at moderate expression levels of *mtrA*, *mtrB* and *mtrF*, the relation between the limiting current density and the arabinose concentration was strikingly linear.

### 9. Decreasing the measurement period

A faster MFC measurement protocol was developed to show that the different induction levels could be sensed without performing a time consuming current-sweep experiment.

Therefore the potential generated by the bacteria was compared at a fixed current density. This experiment was performed using inductor concentrations of 0, 0.5, and 1 mM. To be closer to a putative application, the experiment was conducted with cells that were not precultured with arabinose as inductor. Consequently, these cells were expected to show a delay in MtrFAB expression, which is consistent with the almost identical progression of the determined potentials of all three setups during the first 0.75 h (Figure 6 A). Beyond that period the potential of the induced setups started to drop whereas that of the non-induced setup was barely affected. After 2 h of chronopotentiometry a difference between all three induction levels was clearly visible. Compared to the non-induced setup those with induction levels of 0.5 and 1 mM arabinose reached a 1.13- and 1.75-fold lower potential, respectively (Figure 6 B). Thereby, the described drops of the potentials seemed to be related to the increasing number of expressed MtrFAB-complexes in this phase. This method provided a preferred approach for a quick and specific detection of so far not measurable compounds.

### 10. Determining arabinose concentration in a sample

Using the characteristic linear relationship (Figure 5 C) obtained by current-ramp-chronopotentiometric measurements of *S*. *oneidensis* MTR-FAB which were grown at different arabinose concentrations, the concentration of arabinose in a given sample is determined. A culture of *S*. *oneidensis* MTR-FAB is cultured and conditioned as described above. After reaching a constant potential, the conditioning phase was terminated and the anode covered with a biofilm of *S*. *oneidensis* MTR-FAB transferred into an anode chamber containing media of an unknown arabinose concentration. To determine the arabinose concentration, the amperage of the culture was measured at +750 mV versus a standard hydrogen electrode. The measured amperage is then used to calculate the limiting current density with respect to the anode surface. Finally, the arabinose concentration is revealed according to the observed limiting current density using the characteristic line.

### References

Bücking C, Popp F, Kerzenmacher S, Gescher J; FEMS Microbiol Lett. 2010 May;306(2):144-51. Epub 2010 Mar 30; Involvement and specificity of Shewanella oneidensis outer membrane cytochromes in the reduction of soluble and solid-phase terminal electron acceptors.
Logan BE, Regan JM; Environ Sci Technol. 2006 Sep 1;40(17):5172-80. Microbial fuel cells--challenges and applications.
EI Sheikh AF, Klotz MG; Environ Microbiol. 2008 Nov;10(11):3026-35. Ammonia-dependent differential regulation of the gene cluster that encodes ammonia monooxygenase in Nitrosococcus oceani ATCC 19707.
Farmer WR, Liao JC; Biotechnol Bioeng. 2001 Dec 5;75(5):504-9. Acetate-inducible protein overexpression from the glnAp2 promoter of Escherichia coli.
Guzman LM, Belin D, Carson MJ, Beckwith J; J Bacteriol. 1995 Jul;177(14):4121-30. Tight regulation, modulation, and high-level expression by vectors containing the arabinose PBAD promoter.
Lee SK, Keasling JD; Appl Environ Microbiol. 2005 Nov;71(11):6856-62. A propionate-inducible expression system for enteric bacteria.
Jensen HM et al.; PNAS. 2010 Nov 9;107(45):19213-19218. Engineering of a synthetic electron conduit in living cell.
Coursolle D and Gralnick JA; Frontiers in Microbiology. 2012 Feb;3:1-11. Reconstruction of extracellular respiratory pathways for iron(III) reduction in *Shewanella oneidensis*strain MR-1.

## Claims

1. A biosensor for detecting a compound comprising
- an electrode system,
- a device for measuring electron transfer in the electrode system, and
- a microorganism having a nucleic acid construct comprising the genes *mtrA, mtrB* and *mtrF*, which transfers electrons to the electrode system if the genes are expressed,
wherein the regulatory element is controlled by the compound and the microorganism is devoid of endogenous outer membrane associates cytochrome genes.

2. The biosensor of claim 1, wherein the compound is selected from the group consisting of a hydrocarbon, a peptide, a carbohydrate, an ammonium compound, a carboxylic acid and a salt thereof.

3. The biosensor of claim 1 or 2, wherein the regulatory element comprises a promoter, an inducible promoter, a repressor, an activator and/or an enhancer.

4. The biosensor of any of claims 1 to 3, wherein the regulatory element comprises an arabinose dependent regulatory element, preferably a P_{BAD} promoter, an ammonium-dependent regulatory element, preferably an amo operon promoter, an acetate-dependent regulatory element, preferably a glnAp2 promoter element or a propionate-dependent regulatory element, preferably a P_{prpB} promoter element.

5. The biosensor of claim 1, wherein the micororganism is a bacterial cell, more preferred an facultative anaerobic bacterial cell.

6. The biosensor of claim 5, wherein the bacterial cell is a *Escherichia coli* cell, a *Shewanella* cell, preferably a *Shewanella oneidensis* cell or a *Geobacter* cell, preferably a *Geobacter sulfurreducens* cell.

7. A method for detecting a compound in a sample comprising
- providing a microorganism having a nucleic acid construct comprising the genes *mtrA, mtrB* and *mtrF* under a regulatory element depending on the compound, the microorganism being devoid of endogenous outer membrane associates cytochrome genes,
- contacting the microorganism with an electrode system such that the microorganism transfers electrons to the anode of the electrode system if the genes are expressed,
- adding a sample to the microorganism, and
- measuring the electron transfer to the electrode system,
wherein the electron transfer to the electrode system indicates the absence, presence and/or concentration of the compound in the sample.

8. The method of claim 7, wherein the sample is a sample of a microbiological process, preferably a fermentation process, more preferred a biogas fermentation process.

9. A biosensor for monitoring a fermentation process comprising at least one bio-electrochemical cell with a culture of a microorganism having a nucleic acid construct comprising the genes *mtrA,* and *mtrB* and *mtrF* under a regulatory element such that the microorganism transfers electrons to an electrode of the bio-electrochemical cell if the genes are expressed, wherein the expression of the genes depends on a product or a substrate of the fermentation process and the microorganism is devoid of endogenous outer membrane associates cytochrome genes.

10. A biosensor for monitoring a fermentation process comprising two bio-electrochemical cells each with a culture of a microorganism having a nucleic acid construct comprising the genes *mtrA, mtrB* and *mtrF* under a regulatory element, such that the microorganism transfers electrons to an electrode of the bio-electrochemical cell if the genes are expressed, wherein the regulatory element of the first microorganism is a propionic acid dependent regulatory element and the regulatory element of the second microorganism is an acetic acid dependent regulatory element and wherein the microorganism is devoid of endogenous outer membrane associates cytochrome genes.

11. Use of a cell for detecting a compound comprising a nucleic acid construct with a coding region and a regulatory element, which controls expression of the coding region depending on the compound, wherein the coding region comprises the genes *mtrA, mtrB* and *mtrF,* wherein the cell is devoid of endogenous outer membrane associates cytochrome genes.

## Patentansprüche

1. Biosensor zum Nachweisen einer Verbindung umfassend
- ein Elektrodensystem,
- eine Vorrichtung zum Messen einer Elektronenübertragung in dem Elektrodensystem, und
- einen Mikroorganismus mit einem Nukleinsäurekonstrukt, das die Gene *mtrA*, *mtrB* und *mtrF* umfasst, welches Elektronen auf das Elektrodensystem überträgt, wenn die Gene exprimiert werden,
wobei das regulatorische Element durch die Verbindung kontrolliert ist und der Mikroorganismus frei von endogenen Genen für mit der äußeren Membran assoziierte Cytochrome ist.

2. Biosensor nach Anspruch 1, wobei die Verbindung aus der Gruppe bestehend aus einem Kohlenwasserstoff, einem Peptid, einem Kohlenhydrat, einer Ammoniumverbindung, einer Carbonsäure und einem Salz davon ausgewählt ist.

3. Biosensor nach Anspruch 1 oder 2, wobei das regulatorische Element einen Promoter, einen induzierbaren Promoter, einen Repressor, einen Aktivator und/oder einen Enhancer umfasst.

4. Biosensor nach einem der Ansprüche 1 bis 3, wobei das regulatorische Element ein arabinoseabhängiges regulatorisches Element, vorzugsweise einen P_{BAD}-Promoter, ein ammoniumabhängiges regulatorisches Element, vorzugsweise einen amo-Operon-Promoter, ein acetatabhängiges regulatorisches Element, vorzugsweise ein glnAp2-Promoterelement, oder ein propionatabhängiges regulatorisches Element, vorzugsweise ein P_{prpB}-Promoterelement umfasst.

5. Biosensor nach Anspruch 1, wobei der Mikroorganismus eine Bakterienzelle, weiter bevorzugt eine fakultativ anaerobe Bakterienzelle ist.

6. Biosensor nach Anspruch 5, wobei die Bakterienzelle eine *Escherichia coli-Zelle,* eine *Shewanella-*Zelle, vorzugsweise eine *Shewanella oneidensis*-Zelle, oder eine *Geobacter*-Zelle, vorzugsweise eine *Geobacter sulfurreducens*-Zelle ist.

7. Verfahren zum Nachweisen einer Verbindung in einer Probe umfassend
- Bereitstellen eines Mikroorganismus mit einem Nukleinsäurekonstrukt, das die Gene *mtrA*, *mtrB* und *mtrF* unter einem regulatorischen Element, das von der Verbindung abhängt, umfasst, wobei der Mikroorganismus frei von endogenen Genen für mit der äußeren Membran assoziierte Cytochrome ist,
- Kontaktieren des Mikroorganismus mit einem Elektrodensystem, so dass der Mikroorganismus Elektronen auf die Anode des Elektrodensystems überträgt, wenn die Gene exprimiert werden,
- Hinzufügen einer Probe zu dem Mikroorganismus, und
- Messen der Elektronenübertragung auf das Elektrodensystem,
wobei die Elektronenübertragung auf das Elektrodensystem die Abwesenheit, die Anwesenheit und/oder die Konzentration der Verbindung in der Probe anzeigt.

8. Verfahren nach Anspruch 7, wobei die Probe eine Probe eines mikrobiologischen Prozesses, vorzugsweise eines Fermentationsprozesses, weiter bevorzugt eines Biogasfermentationsprozesses ist.

9. Biosensor zum Überwachen eines Fermentationsprozesses umfassend mindestens eine bioelektrochemische Zelle mit einer Kultur eines Mikroorganismus mit einem Nukleinsäurekonstrukt, das die Gene *mtrA*, und *mtrB* und *mtrF* unter einem regulatorischen Element umfasst, so dass der Mikroorganismus Elektronen auf eine Elektrode der bioelektrochemischen Zelle überträgt, wenn die Gene exprimiert werden, wobei die Expression der Gene von einem Produkt oder einem Substrat des Fermentationsprozesses abhängt und der Mikroorganismus frei von endogenen Genen für mit der äußeren Membran assoziierte Cytochrome ist.

10. Biosensor zum Überwachen eines Fermentationsprozesses umfassend zwei bioelektrochemische Zellen, jede mit einer Kultur eines Mikroorganismus mit einem Nukleinsäurekonstrukt, das die Gene *mtrA*, *mtrB* und *mtrF* unter einem regulatorischen Element umfasst, so dass der Mikroorganismus Elektronen auf eine Elektrode der bioelektrochemischen Zelle überträgt, wenn die Gene exprimiert werden, wobei das regulatorische Element des ersten Mikroorganismus ein propionsäureabhängiges regulatorisches Element ist und das regulatorische Element des zweiten Mikroorganismus ein essigsäureabhängiges regulatorisches Element ist und wobei der Mikroorganismus frei von endogenen Genen für mit der äußeren Membran assoziierte Cytochrome ist.

11. Verwendung einer Zelle zum Nachweisen einer Verbindung umfassend ein Nukleinsäurekonstrukt mit einer kodierenden Region und einem regulatorischen Element, welches die Expression der kodierenden Region abhängig von der Verbindung kontrolliert, wobei die kodierende Region die Gene *mtrA*, *mtrB* und *mtrF* umfasst, wobei die Zelle frei von endogenen Genen für mit der äußeren Membran assoziierte Cytochrome ist.

## Revendications

1. Biocapteur destiné à détecter un composé comprenant
- un système d'électrodes,
- un dispositif destiné à mesurer un transfert d'électrons dans le système d'électrodes, et
- un micro-organisme ayant une chimère d'acide nucléique comprenant les gènes *mtrA*, *mtrB* et *mtrF*, qui transfère des électrons au système d'électrodes si les gènes sont exprimés,
dans lequel l'élément régulateur est contrôlé par le composé et le micro-organisme est dénué de gènes de cytochrome associés à la membrane externe endogène.

2. Biocapteur selon la revendication 1, dans lequel le composé est sélectionné dans le groupe constitué par un hydrocarbure, un peptide, un glucide, un composé d'ammonium, un acide carboxylique et un sel de celui-ci.

3. Biocapteur selon la revendication 1 ou 2, dans lequel l'élément régulateur comprend un promoteur, un promoteur inductible, un répresseur, un activateur et/ou un amplificateur.

4. Biocapteur selon l'une quelconque des revendications 1 à 3, dans lequel l'élément régulateur comprend un élément régulateur dépendant de l'arabinose, de préférence un promoteur P_{BAD}, un élément régulateur dépendant de l'ammonium, de préférence un promoteur de l'opéron amo, un élément régulateur dépendant de l'acétate, de préférence un élément du promoteur glnAp2 ou un élément régulateur dépendant du propionate, de préférence un élément du promoteur P_{prpB}.

5. Biocapteur selon la revendication 1, dans lequel le micro-organisme est une cellule bactérienne, plus préférablement une cellule bactérienne anaérobie facultative.

6. Biocapteur selon la revendication 5, dans lequel la cellule bactérienne est une cellule d'*Escherichia coli,* une cellule de *Shewanella,* de préférence une cellule de *Shewanella oneidensis,* ou une cellule de *Geobacter,* de préférence une cellule de *Geobacter sulfurreducens.*

7. Méthode destinée à détecter un composé dans un échantillon comprenant
- la fourniture d'un micro-organisme ayant une chimère d'acide nucléique comprenant les gènes *mtrA*, *mtrB* et *mtrF* sous un élément régulateur dépendant du composant, le micro-organisme étant dénué de gènes de cytochrome associés à la membrane externe endogène,
- la mise en contact du micro-organisme avec un système d'électrodes de telle sorte que le micro-organisme transfère des électrons à l'anode du système d'électrodes si les gènes sont exprimés,
- l'ajout d'un échantillon au micro-organisme, et
- la mesure du transfert d'électrons au système d'électrodes,
dans laquelle le transfert d'électrons au système d'électrodes indique l'absence, la présence et/ou la concentration du composé dans l'échantillon.

8. Méthode selon la revendication 7, dans laquelle l'échantillon est un échantillon d'un procédé microbiologique, de préférence un procédé de fermentation, plus préférablement un procédé de fermentation de biogaz.

9. Biocapteur destiné à la surveillance d'un procédé de fermentation comprenant au moins une cellule bioélectrochimique avec une culture d'un micro-organisme ayant une chimère d'acide nucléique comprenant les gènes *mtrA* et *mtrB* et *mtrF* sous un élément régulateur de telle sorte que le micro-organisme transfère des électrons à une électrode de la cellule bioélectrochimique si les gènes sont exprimés, dans lequel l'expression des gènes dépend d'un produit ou d'un substrat du procédé de fermentation et le micro-organisme est dénué de gènes de cytochrome associés à la membrane externe endogène.

10. Biocapteur destiné à la surveillance d'un procédé de fermentation comprenant deux cellules bioélectrochimiques chacune avec une culture d'un micro-organisme ayant une chimère d'acide nucléique comprenant les gènes *mtrA*, *mtrB* et *mtrF* sous un élément régulateur de telle sorte que le micro-organisme transfère des électrons à une électrode de la cellule bioélectrochimique si les gènes sont exprimés, dans lequel l'élément régulateur du premier micro-organisme est un élément régulateur dépendant de l'acide propionique et l'élément régulateur du deuxième micro-organisme est un élément régulateur dépendant de l'acide acétique et dans lequel le micro-organisme est dénué de gènes de cytochrome associés à la membrane externe endogène.

11. Utilisation d'une cellule destinée à détecter un composé comprenant une chimère d'acide nucléique avec une région codante et un élément régulateur, qui contrôle l'expression de la région codante dépendant du composé, dans laquelle la région codante comprend les gènes *mtrA*, *mtrB* et *mtrF*, dans laquelle la cellule est dénuée de gènes de cytochrome associés à la membrane externe endogène.
